# EUROPEAN PATENT SPECIFICATION

(11) **EP 2 961 366 B1**
(45) Date of publication and mention of the grant of the patent: **18.09.2019**
(21) Application number: 14728621.5
(22) Date of filing: 25.04.2014
(51) Int. Cl.: A61F 13/08, A61F 5/01

(54) **COMPRESSION DEVICE**
KOMPRESSIONSVORRICHTUNG
DISPOSITIF DE COMPRESSION

(30) Priority: 27.02.2013 GB 201303431; 20.03.2013 GB 201305081; 26.09.2013 GB 201317061
(43) Date of publication of application: 06.01.2016
(73) Proprietor: 3M Innovative Properties Company, St. Paul, MN 55133-3427 (US)
(72) Inventor: HITSCHMANN, Guido, 41453 Neuss (DE)
(74) Representative: Gabriel, Kiroubagaranne
(86) International application number: PCT/IB2014/000613
(87) International publication number: WO 2014/132127

(56) References cited:
- EP-A1- 1 459 713
- WO-A1-01/72250
- DE-A1- 4 419 287
- DE-U1-202008 008 422
- US-A- 5 918 602
- US-A1- 2005 288 614
- US-A1- 2011 125 183
- US-B1- 6 254 554

## Description

### Field

The present invention relates to compression devices, in particular compression devices for applying compression to a portion of a limb of a user for the use in the treatment and/or management of oedema and other venous and lymphatic disorders of a limb, more particularly venous leg ulcers and lymphoedema of a limb.

### Background

Compression therapy is generally prescribed to support an insufficient venous or lymphatic system in returning blood or lymph to the heart. Accordingly compression is generally considered to be the standard treatment for use in the treatment of oedema and other venous and lymphatic disorders e.g. of the lower limbs venous leg ulcers and other clinical conditions such as lymphoedema. The positive effects of compression therapy on venous lymph return as well as on the healing of chronic venous (leg) ulcers are well documented in the medical literature.

Compression bandages and stockings are the most common compression systems used for compression therapy. Compression stockings however often do not provide the desired therapeutic compressive pressure, since such stockings generally need to be quite elastic so that one can pull them on and off. Compression bandages, being made of materials having much lower elastic (or even in some cases nearly non-elastic) characteristics, are typically much more effective in supporting the muscle pump to return venous blood to the heart and thus in compression therapy compared to compression stockings. Compression bandages however typically need to be applied by a well-trained professional in order to achieve the desired and/or needed pressure profile, and such bandages typically need to be reapplied frequently due to change (e.g. reduction) in the volume of the limb (e.g. as a result of oedema reduction).

A large number of other compression systems have been proposed. A number of these can be generically described as to include a garment to be wrapped around a limb and a closure mechanism to secure the garment around the limb (e.g. see US 3,538,194, US 3,856,008, US 3,845,769, US 5,653,244, WO 01/72250, US 2002/0062096, US 2003/195449, US 2005/0192524, WO 2006/048619). Although quite of number of non-bandage-type and non-stocking-type compression systems have been proposed, only a few have been commercialized; examples include products marketed under the trade designation FARROWWRAP, JUXTA-FIT and JUXTA-CURES. Notably these commercial products although given as alternatives to typical compression bandaging systems include multiple band-like elements for winding and wrapping (e.g. overlapping and/or cross winding/wrapping).

### Summary of Invention

There is an ongoing need for non-bandage and non-stocking type compression devices that are easy to use and to apply, desirably by non-trained personnel or even the patient, while at the same time facilitating a recognition of whether the desired and/or needed proper fit has been achieved and/or maintained, which in turns facilitates the provision of desirable uniformity of compression.

In one aspect of the present invention there is provided a compression device for applying compression to a limb of a user comprising a sleeve for substantially covering a portion of the limb of a user, wherein the sleeve has an outer surface, an upper edge, a lower edge and two lateral side edges, wherein in the transverse direction from the first lateral side edge to the second lateral side edge the sleeve comprises a first lateral side region, a central region and a second lateral side region, wherein the second lateral edge region of the sleeve is provided with a plurality of rings in series between the upper and lower edges of the sleeve, each ring being releasably or fixedly attached by a strap extending between the sleeve and the ring in substantially the transverse direction of the sleeve, at least a portion of said strap being expandable in at least the transverse direction, wherein said expandable portion comprises a material having elasticity in at least the transverse direction of the sleeve and is configured and arranged, such that when the expandable portion is in its non-expanded state there is exteriorly a loop of material rising outwardly and when, in use under the provision of tension in the transverse direction of the sleeve, the expandable portion expands in the transverse direction and the loop flattens;
wherein the device further comprises a plurality of strip-shaped mechanical fastening tabs, wherein a single tab is provided for each ring, each tab comprising a proximal end portion and a distal end portion being connected by an inner tab portion, wherein said proximal end portion is releasably or fixedly attached to the first lateral edge region of sleeve such that the tab is located opposite to a ring and extends in substantially the transverse direction of the sleeve, with its distal end portion positioned away from the central portion of the sleeve, wherein each tab has a first major surface located towards the outer surface of the sleeve and a second major surface located away from the outer surface of the sleeve, wherein the second major surface at the distal end portion of the tab comprises one part of a mechanical fastening system and said second major surface at the proximal end portion of the tab comprise the complementary part of the mechanical fastening system; and wherein the tabs and rings are configured and arranged such that, in use, the tabs are passed through the rings, turned back on themselves such that the first lateral side edge of the sleeve is drawn towards the rings and then fastened so that the sleeve is tightened and restrained about the limb of the user.

In another aspect of the present invention there is provided a compression device for applying compression to a limb of a user comprising a sleeve for substantially covering a portion of the limb of a user, wherein the sleeve has an outer surface, an upper edge, a lower edge and two lateral side edges, wherein in the transverse direction from the first lateral side edge to the second lateral side edge the sleeve comprises a first lateral side region, a central region and a second lateral side region, wherein the second lateral edge region of the sleeve is provided with either a plurality of eyelets or a plurality of rings in series between the upper and lower edges of the sleeve, wherein the sleeve includes an elongate, expandable gusset extending substantially lengthwise between the upper and lower edges of the sleeve, said gusset being expandable in at least the transverse direction of the sleeve, wherein said expandable gusset comprises a material having elasticity in at least the transverse direction and is configured and arranged, such that when the expandable gusset is in its non-expanded state there is exteriorly a loop of material rising outwardly, and when, in use under the provision of tension in the transverse direction of the sleeve, the expandable gusset expands in the transverse direction and the loop flattens;
wherein the device further comprises a plurality of strip-shaped mechanical fastening tabs, wherein a single tab is provided for each eyelet or ring, as applicable, each tab comprising a proximal end portion and a distal end portion being connected by an inner tab portion, wherein said proximal end portion is releasably or fixedly attached to the first lateral edge region of sleeve such that the tab is located opposite to a eyelet or ring, as applicable, and extends in substantially the transverse direction of the sleeve with its distal end portion positioned away from the central portion of the sleeve, wherein each tab has a first major surface located towards the outer surface of the sleeve and a second major surface located away from the outer surface of the sleeve, wherein the second major surface at the distal end portion of the tab comprises one part of a mechanical fastening system and said second major surface at the proximal end portion of the tab comprise the complementary part of the mechanical fastening system; and wherein the tabs and eyelets or rings, as applicable, are configured and arranged such that, in use, the tabs are passed through the eyelets or rings, as applicable, turned back on themselves such that the first lateral side edge of the sleeve is drawn towards the eyelets or rings, as applicable, and then fastened so that the sleeve is tightened and restrained about the limb of the user.

For the sake of clarity, it is to be appreciated that after application of a compression device onto a limb of a user, the transverse direction of the sleeve will also be a circumferential direction. In accordance with ASTM D4848-98 (2012) and BS EN 14704-1:2005 elasticity is that property of a material by virtue of which it tends to recover its original size and shape immediately after removal of the force causing deformation.

Surprisingly it has been found that by providing a sleeve-like garment that is to be opened and closed with a releasable, mechanical closure system including fastening tabs provided with respective complementary parts of the mechanical closure system in conjunction with opposing rings or eyelets together with an expandable strap portion or an expandable gusset comprising a material having elasticity in at least the transverse direction and being configured and arranged such that, when the expandable strap portion or gusset, respectively, is in its non-expanded state (e.g. when the compression device is not in use) there is a loop of material to the exterior and rising outwardly and when, in use under the provision of tension in the transverse direction of the sleeve, the expandable strap portion or gusset, respectively, expands in the transverse direction and the loop flattens (eventually disappearing), it is possible to provide compression devices which are easy to use and apply where the person applying the device is also advantageously provided with an visual indication facilitating an assessment towards the extent of extension and the provision of a good anatomic fit. Moreover, when there is no extension or only partial extension of the expandable strap portion or gusset, the outwardly facing loop will be fully raised or only partly flatten, and thus visible as such, and when there is full extension of the expandable strap portion or gusset the outwardly facing loop will disappear (i.e. it will flatten to such an extent there is no longer a loop of material rising outwardly). Such a visual indication is advantageous during the application because once the loop fully flattens out (and thus disappears) there is full extension and thus an indication towards sufficient anatomical fit. Moreover, by providing such a loop-indicating configuration in the straps of the plurality of rings that are provided in series between the upper and lower edges of the sleeve or in the elongate, expandable gusset that extends substantially lengthwise between the upper and lower edges of the sleeve, it is possible to have a visual indication towards extent of extension and thus fit over respective height of the sleeve between its upper and lower edges, so that if desired and/or needed, the extent of tightening of an individual fastening tab threaded through its opposing ring or eyelet can adjusted facilitating the provision of a desirable anatomic fit over the portion of the limb of a user covered by the sleeve and thus in turn facilitating uniformity of compression. The loop-indicating configuration is also favorably useful while the user is wearing the compression device. For example, if, as in fact is desired, the volume of the limb is reduced for example as a result of oedema reduction due to effective compression therapy, the extent of tension on the device and on the expandable strap-portion or gusset will be reduced and the previously flattened loop will then noticeably pucker outwardly forming a fully raised or somewhat flattened loop, depending on the extent of reduced tension and thus providing an indication that the device should be re-tightened or re-applied.

Desirably the expandable strap portion or the gusset, as applicable, comprises two layers, an outer (outwardly facing) layer of material and an inner (inwardly facing) layer of material, wherein the product of the modulus of elasticity of the inner-layer-material times the thickness of the inner-layer-material is less than the product of the modulus of elasticity of the outer-layer-material times the thickness of the outer-layer-material, wherein outer-layer-material is the loop material, and wherein the inner layer of material is affixed to the outer layer of material, so as to provide a loop of outer-layer-material above the inner layer when the expandable strap portion or gusset, respectively, is in its non-expanded state, which in use under the provision of tension and accordingly expansion of expandable strap portion or gusset in the transverse direction, respectively, can flatten. It has been found favorable that in the loop-indicating configuration, the product of the modulus of elasticity of the inner-layer-material times the thickness of the inner-layer-material is at least a factor of two times, in particular at least a factor of four times, lower than the product of the modulus of elasticity of the outer-layer-material times the thickness of the outer-layer-material, so that the transition from a pronounced loop to a completely flattened out loop does not require too much elongation in terms of length. Favorably at least one of the outer-layer- and inner-layer-materials has elasticity in at least the transverse direction, more favorably each said material having elasticity in at least the transverse direction.

Modulus of elasticity (also called elastic modulus) may be determined in accordance to ASTM D 882-09 entitled "Standard Test Method for Tensile Procedure of Thin Plastic Sheeting". It is to be noted that although the standard expressly states that it covers the determination of tensile properties of plastics in the form of thin sheeting, it has been found that the described test method and determination of modulus of elasticity may be suitably used in regard to materials suitable for use in compression devices described herein.

The sleeve, in particular at least the central region of the sleeve, favorably comprises a material that is suitable for use in applying compression, more favorably at least the central region of the sleeve comprises a material having elasticity and a maximum stretch greater than 0% up to 60% in at least the transverse direction of the sleeve under a load of 10 N per cm width e.g. as measured in accordance with DIN 61632:2009. Herein such a material is referred to as a "short-stretch material". Referring to the textbook Foeldi's Textbook of Lymphology, 2nd Edition, extensibility, in other words elasticity, of compression bandages include short stretch (those having a maximum amount of stretch under a load of 10 N per cm width of up to 60%, i.e. greater than 0% and up to 60%); moderate-stretch (60 to 140%) and long stretch (greater than 140%), where correspondingly non-elastic bandages are understood to have no amount of (i.e. 0%) stretch.

To further underline the indicating effect of the loop- indicating configuration and/or to further facilitate the provision of uniformity of compression, the product of modulus of elasticity of the material of the loop (e.g. the outer-layer-material in the two-layer expandable strap portion/gusset embodiments) times the thickness of the material of the loop is favorably at least 90% of the product of the modulus of elasticity of said short-stretch material times the thickness of said short-stretch material, in particular the product of the modulus of elasticity of the material of the loop (e.g. the outer-layer-material in the two-layer expandable strap portion/gusset embodiments) times the thickness of the material of the loop is equal to or greater than the product of the modulus of elasticity of said short-stretch material times the thickness of said short-stretch material.

For ease in viewing the loop-indicating configuration, the expandable portion of the strap or gusset favorably has in its non-expanded state a width relative to the transverse direction of the sleeve of at least 0.1 cm. in particular 0.5 cm. To facilitate favorably sleeve construction e.g. in terms of sizing of components and material use, desirably the expandable portion of the strap or gusset has in its non-expanded state a width relative to the transverse direction of the sleeve of at most 4 cm, in particular at most 3 cm:

Favorably, especially in regard to facilitating viewing of changes in and flattening of the loop, the expandable portion of the strap or gusset has in its expanded state at the point where the loop just fully flattens out a width relative to the transverse direction of the sleeve of at least 1 cm. For ease in viewing and/or to facilitate uniformity of compression in the transverse/ circumferential direction, the expandable portion of the strap or gusset favorably has in its expanded state at the point where the loop just fully flattens out a width relative to the transverse direction of the sleeve is at most 8 cm, in particular at most 6 cm.

To further underline the indicating effect of the expandable strap portion or gusset in two-layer expandable strap portion/gusset embodiments, the inner layer of material and outer layer of material can be favorably configured and arranged, so as to provide at least one loop of outer-layer-material above the inner layer when the expandable strap portion/gusset is in its non-expanded state which, in use under the provision of tension and accordingly expansion of expandable strap portion /gusset in the transverse direction, can flatten and additionally at least one region of outer-layer material above the inner layer, where the outer-layer material includes a slit (e.g. an opening) extending substantially lengthwise relative to said upper and lower edges of the sleeve (termed in the following as "slit-region"), so that when the expandable strap portion/gusset is in its non-expanded state, the outer-layer material in said at least one slit-region lies substantially flat with the lateral edges of said slit near to or overlapping one another and in use, under the provision of tension and accordingly expansion of expandable strap portion/gusset in the transverse direction, the lateral edges of the said slit move apart exposing the underlying inner layer. It will appreciated that the slit-region acts like a window or eye, that is "closed" when the expandable strap portion/gusset is in its non-expanded state covering the underlying inner layer, and "opens" when the expandable strap portion/gusset expands in the transverse direction, and thus providing a second indication towards proper expansion and tensioning of the sleeve. The indicating effect can be further enhanced by providing the inner layer (in particular at least the upper surface thereof) with indicia that become visible when the edges of the slit move apart and/or a color that different to the color of the outer-layer, in particular different to the color of the slit region of the outer-layer. Desirably the at least one loop of outer-layer material and said at least one slit-region are provided in series lengthwise relative to said upper and lower edges of the sleeve, more desirably at least two loops of outer-layer material are provided, said at least two loops of outer-layer material and said at least one slit-region being provided alternatively and in series lengthwise relative to said upper and lower edges of the sleeve.

In regard to the first aspect (i.e. embodiments including an expandable strap portion), to further facilitate the indication and provision of a favorable anatomic fit over the portion of the limb (e.g. the lower leg including the calf) of a user that is covered by the sleeve in use, favorably the plurality of rings or straps including the interstices between rings or straps, respectively extends over a height corresponding to at least 70% of the height of the sleeve from the upper to lower edge. Generally it is desirable that the plurality of rings or straps including the interstices between rings or straps, respectively extends a height corresponding from 70% up to and including 100% , and possibly greater than 100% of the height of the sleeve from the upper to lower edge. The height of the interstices between rings or straps may range from 0.1 mm to 7 cm, inclusive, in particular from 0.3 mm to 3 cm, inclusive, and more particularly from 0.5 mm to 2 cm.

In regard to the second aspect (i.e. embodiments including a gusset), to further facilitate the indication and provision of a favorable anatomic fit over the portion of the limb (e.g. the lower leg including the calf) of a user that is covered by the sleeve in use, favorably the gusset extends a height corresponding to 70% up to and including 100% of the height of the sleeve from the upper to lower edge. Also to further facilitate provision of favorable anatomic fit it is desirable that the plurality of eyelets or rings, as applicable, including the interstices between eyelets or rings, respectively extends over a height corresponding to at least 70% of the height of the sleeve from the upper to lower edge. Generally it is desirable that the plurality of eyelets or rings, as applicable, including the interstices between eyelets or rings, respectively extends a height corresponding from 70% up to and including 100% of the height of the sleeve from the upper to lower edge. The height of the interstices between eyelets and rings, as applicable, may range from 0.1 mm to 7 cm, inclusive, in particular from 0.3 mm to 3 cm, inclusive, and more particular from 0.5 mm to 2 cm.

The above summary of the present invention is not intended to describe each disclosed embodiment or every implementation of the present invention. The description that follows more particularly exemplifies illustrative embodiments. Also further embodiments are described in dependent claims. In several places throughout the application, guidance is provided through lists of examples, which examples can be used individually and in various combinations. In each instance, the recited list serves only as a representative group and should not be interpreted as an exclusive list.

### Brief Description of Drawings

The invention will now be described with reference to the accompanying drawings in which:
Figure 1 represents a top view of an exemplary embodiment of a compression device in accordance with the first aspect of the invention described herein, while Figure 2 shows a cross-sectional view of the exemplary embodiment depicted in Figure 1.
Figure 3a represents a perspective, front view of the exemplary embodiment depicted in Figures 1 and 2 shown in use on the lower leg of a user, while Figure 3b shows a projection of the axis R (which is depicted in Figure 3a) onto a plane P containing the central axis A (that is also depicted in Figure 3a).
Figure 4 represents a top view of another exemplary embodiment of a compression device in accordance with the first aspect of the invention described herein, while Figure 5 shows a cross-sectional view of the exemplary embodiment in Figure 4.
Figure 6 represents a top view of an exemplary embodiment of a compression device in accordance with the second aspect of the invention described herein, while Figure 7 shows a cross-sectional view of the exemplary embodiment in Figure 6.
Figure 8 represents a top view of another exemplary embodiment of a compression device in accordance with the second aspect of the invention described herein, while Figure 9 shows a cross-sectional view of the exemplary embodiment in Figure 8.
Figure 10a shows a perspective, front view of the exemplary embodiment in depicted in Figures 8 and 9 in use on the lower leg of a user and Figure 10b shows a projection of the axes G and E (which are depicted in Figure 10a) onto a plane P containing the central axis A (that is also depicted in Figure 10a).
Figure 11 represents a top view of another exemplary embodiment of a compression device in accordance with the second aspect of the invention described herein, while Figure 12 shows a cross-sectional view of the exemplary embodiment in Figure 11.
Figure 13 represents a top view of an exemplary embodiment of a compression device in accordance with the first aspect of the invention described herein, while Figures 14 and 15 shows two cross-sectional views of the exemplary embodiment depicted in Figure 13.
Figure 16 represents a perspective, front view of the exemplary embodiment depicted in Figures 13 to 15 shown in use on the lower leg of a user.

In the description that follows, unless expressly stated otherwise, terms such as 'top', 'bottom', 'above', 'below', etc, refer only to features as shown in the Figures, and no restriction as to orientation of use, etc, is intended. Not all Figures are to the same scale.

### Detailed Description

It is to be understood that the present invention covers all combinations of particular, suitable, desirable, favorable, advantageous and preferred aspects of the invention described herein.

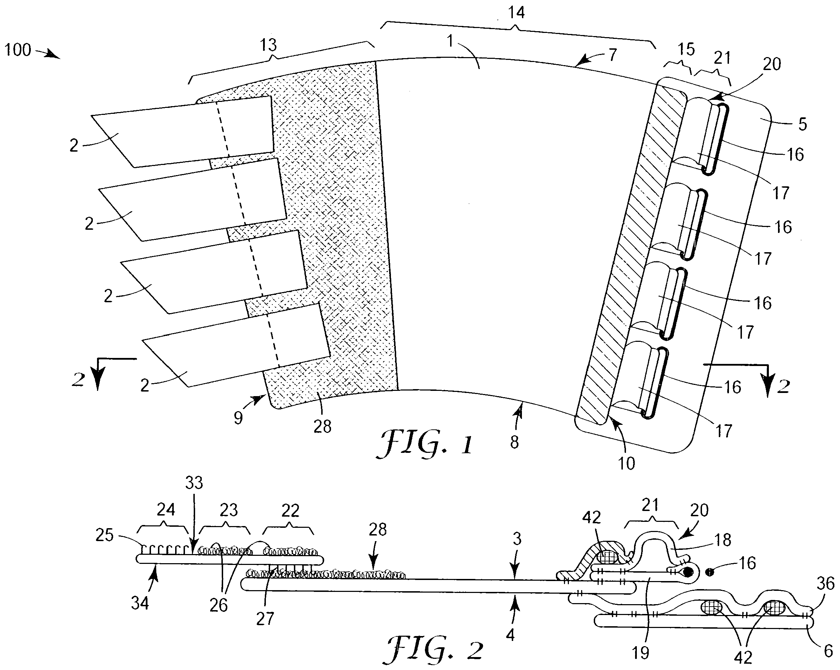

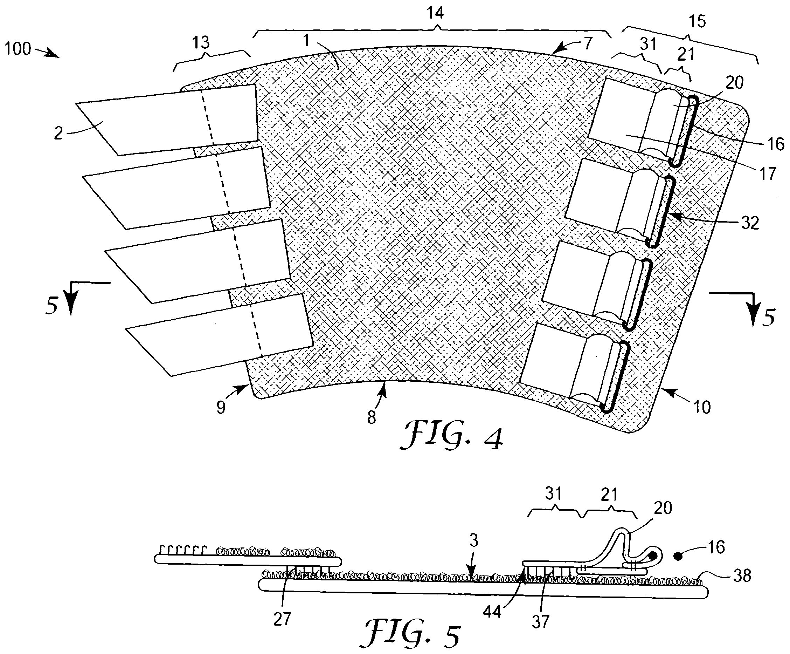

Figure 1 shows a top view of the exterior of an exemplary embodiment of a compression device (100) for use in applying compression to a limb of a user, while Figure 2 shows a cross-sectional view of this exemplary embodiment. The device comprises a sleeve (1) for substantially covering a portion of the limb of a user. The sleeve includes an outer surface (3), an inner surface (4), an upper edge (7) and a lower edge (8). When the device is in use on the limb, typically the inner surface (4) is located towards the wearer/user (in the following the term "inner" will typically refer to something located towards the wearer/user and "outer" away from the wearer/user), while the upper edge is located towards to the torso of the user and the lower edge distant to the torso of the user, and both upper and lower edges, being essentially transverse, will be located essentially circumferentially around the limb after application. As can be appreciated from Figure 1, the sleeve includes two lateral side edges (9, 10) extending from its upper edge to its lower edge. In the transverse direction from the first lateral side edge (9) to the second lateral side edge (10) the sleeve comprises a first lateral side region (13), a central region (14) and a second lateral side region (15). The second lateral edge region of the sleeve is provided with a plurality of rings (16) in series between the upper and lower edges of the sleeve. Each ring is attached, in this particular exemplary embodiment fixedly attached, by a strap (17) that extends in substantially the transverse direction of the sleeve between the sleeve, in particular the second lateral side region and the ring. As mentioned above, it is to be recognized that after application of a compression device onto a limb of a user, the transverse direction of the sleeve will also be a circumferential direction. Referring to the cross-sectional view in Figure 2, it can be seen that the strap (17) is attached to the second lateral side region (15) of the sleeve (1), in particular onto the outer surface (3) of the sleeve at the second lateral side region. In this exemplary embodiment, straps are fixedly attached, however it is to be appreciated that in alternative embodiments (e.g.as shown in the exemplary depicted in Figures 4 and 5) straps could be releasably attached.

Straps comprise an expandable strap portion comprising a material having elasticity in at least the transverse direction and being configured and arranged such that when the expandable strap portion is in its non-expanded state (e.g. when the compression device is not in use) there is exteriorly a loop of material rising outwardly and when, in use under the provision of tension in the transverse direction of the sleeve, the expandable strap portion expands in the transverse direction and the loop flattens (eventually disappearing).

Returning to the exemplary embodiment depicted in Figures 1 and 2, it can be seen that each strap (17) include a expandable portion (21) that is configured with a loop (20) towards the exterior and rising outwardly. This is best seen in cross-sectional view shown in Figure 2 which like Figure 1 shows the expandable strap portion in its non-expanded state. It can also be seen that the expandable portion of the strap favorably comprises two layers, an outer layer of material (18) and an inner layer of material (19) and wherein the inner layer of material is affixed to the outer layer of material, so as to provide a loop of outer-layer-material (i.e. loop (20)) above the inner layer. At least one of (desirably both) the inner-layer-material and outer-layer-material has (have) elasticity in at least the transverse direction. As a result of inner and/or outer-layer material properties, the configuration of the attachment of the inner and outer-layer-materials to one another as well as the configuration of the attachment of the strap to the ring and onto the sleeve, the loop-containing portion (21) of the strap is expandable in at least the transverse direction. When the expandable portion of the strap is not expanded, i.e. in its non-expanded state, as shown in Figures 1 and 2, the loop (20) is visible as an elongate mound or hump. In use, when the device is applied onto the limb of a user, tension will be provided and accordingly the expandable portion (21) of the strap will expand in the transverse direction and the loop can and will flatten.

For those embodiments where the expandable portion of the strap includes two layers, as indicated above, favorably the product of the modulus of elasticity (in the transverse direction) of the inner-layer-material times the thickness of the inner-layer material is less than the product of the modulus of elasticity (in the transverse direction) of the outer-layer-material times the thickness of the outer-layer material. More favorably, the product of the modulus of elasticity of the inner-layer-material times the thickness of the inner-layer-material is at least a factor of two times, more favorably at least a factor of four times, lower the product of the modulus of elasticity of the outer-layer-material times the thickness of the outer-layer-material.

In the exemplary embodiment of Figures 1 and 2, when the expandable portions of the straps are in their non-expanded state, the rings are positioned along and spaced apart from the second lateral edge of the sleeve away from the second lateral edge portion. In particular, the lateral edge of each ring that is near to the second lateral edge of the sleeve is spaced apart from the second lateral edge at a distance of at most 4 cm, in particular at most 3 cm. Alternatively, when the expandable portions of the straps are in their non-expanded state, the rings could be positioned directly adjacent to second lateral edge, in particular the lateral edge near the second lateral edge of the sleeve could be positioned directly adjacent to second lateral edge of the sleeve.

Sleeves, when laid out flat e.g. as depicted in Figure 1, may be substantially rectangular, trapezoidal or irregular in shape. For example, the sleeves in a number of exemplary embodiments depicted herein (e.g. in Figure 1) are substantially trapezoidal in shape. For facilitating an optimal fit onto a part of the limb of a user, the upper edge and/or the lower edge of the sleeve may be favorably slightly curved, in particular the upper edge may be slightly convex and/or the lower edge which is normally positioned distant to the torso of the user, may be either slightly concave or convex. Alternatively or in addition thereto, one or both of the lateral side edges may be slightly curved, in particular slightly convex. This may be facilitating fitting over well-developed calves. In use, when the compression device is applied onto a limb of the user, favorably the sleeve is substantially cylindrical, barrel or truncated-conical in shape.

The compression device further comprises a plurality of strip-shaped mechanical fastening tabs (2). There is a single tab provided for each ring (16). Each tab comprises a proximal end portion (22) and a distal end portion (24) being connected by an inner tab portion (23). In the exemplary embodiment shown here in Figures 1 and 2 as well as the other exemplary embodiments described herein, the proximal end portion (22) is releasably attached to the first lateral edge region (13) of sleeve (1). It is to be appreciated that the proximal end portion could alternatively being fixedly attached (e.g. via adhesive, bonding, or stitching) to the first lateral edge region of the sleeve. The fastening tabs (2) are attached onto the sleeve such that there is a tab located opposite to a ring and such that each tab extends in substantially the transverse direction of the sleeve, with its distal end portion (24) positioned away from the central portion of the sleeve. From Figure 2, it can be seen that each tab has a first major surface, i.e. an inner surface, (34) located towards the outer surface (3) of the sleeve and a second major surface, i.e. an outer surface, (33) located away from the outer surface of the sleeve. The second major surface (33) at the distal end portion (24) of the tab comprises one part (25) of a mechanical fastening system and said second major surface at the proximal end portion of the tab comprises the complementary part (26) of the mechanical fastening system. As can be seen in this exemplary embodiment shown in Figures 1 and 2 and the other exemplary embodiments described herein, the second major surface at the inner tab portion of the fastening tab may also comprise the complementary part (26) of the mechanical fastening system.

In general, fastening tabs are favorably configured such that the second major surface at the distal end portion of the fastening tabs is provided with hook, stem and/or cup-shaped fasteners (first tab fasteners) and the second major surface at the proximal end portion of the fastening tabs has a structure or is provided with a structure that is adapted to be engaged by said first tab fasteners. The second major surface at the inner portion of the fastening tabs may also have a structure or been provided with a structure that is adapted to be engaged by the first tab fasteners.

As indicated above, the fastening tabs may be either releasably or fixed attached to the sleeve. Favorably the fastening tabs are attached such that the tabs extend across the first lateral side edge. In particular the tabs generally extend at least 2 cm outwardly beyond the first lateral side edge. For those embodiments in which the fastening tabs are releasably attached, favorably the first major surface at the proximal end portion of the fastening tabs is provided with hook, stem and/or cup-shaped fasteners (second tab fasteners) and the outer surface at the first lateral edge region of the sleeve has a structure or is provided with a structure that is adapted to be engaged by said second tab fasteners. Here the first major surface at the proximal end portion of the fastening tabs can then be releasably attached to the outer surface at the first lateral edge region of sleeve. The second tab fasteners may be identical to the first tab fasteners or different. In the event that the second and first tab fasteners are different, favorably the outer surface at the first lateral edge region of the sleeve has a structure or is provided with a structure that is adapted to be engaged by both the first and second fasteners. Favorably, the second tab fasteners and the relevant complementary engagement structure are selected such that they provide a stronger fastening than the first tab fasteners and the relevant complementary engagement structure. The use of releasably attachable fastening tabs can be particularly useful in that it can allow for the provision of compression devices that can be adjusted in their width.

Compression devices described herein, in particular the sleeves thereof, can be provided in different sizes to accommodate the difference in the size of limbs (e.g. arms versus legs) as well as the general difference in sizes of a particular limb. Compression devices described herein are particularly suitable for use on the lower leg including the calf. In regard to the latter, for example considering the size of an adult human lower leg, including those persons suffering from lymphodemia, can range from around 130 to 420 mm in circumference at the ankle and around from 280 to 650 mm in circumference at their widest point, it could be possible to provide compression devices in for example seven standard (width) sizes, e.g. XS, S; M, L, XL, XXL, XXXL, aimed to cover 80% of the potential relevant circumferential sizes of the potential users while the remaining 20% could be provided for by special order. In addition, considering the length of an adult human lower leg can range from around 20 cm to 40 cm, it could be possible to provide in conjunction with the standard (width) sizes mentioned above, three height sizes, e.g. short, average and, tall, again aimed to cover 80% of the potential relevant lengths of the potential users. In regard to the standard width sizes, the number of standard sizes to cover 80% the potential relevant circumferential sizes of the potential users could be reduced by for example providing compression devices configured such that the width of the sleeve could be readily adjusted by the user or the care-giver applying the compression device onto the limb of the user. In particular, it would be advantageous to provide compression devices wherein the fastening tabs is releasably attachable to the first laterial edge region of the sleeve as described herein and wherein the first lateral edge region of the sleeve is configured such that it is trimmable.

Returning to the exemplary embodiment depicted in the Figures 1 and 2, it can be seen that the second major (outer) surface (33) at the distal end portion (24) of each fastening tab (2) is provided with hook, stem and/or cup-shaped fasteners (25) and the second major (outer) surface at the proximal end portion (22) as well as at the inner tab portion (22) of the fastening tabs has a structure or is provided with a structure (26) that is adapted to be engaged by said tab fasteners. In addition, the first major (inner) surface (34) at the proximal end portion (22) of each fastening tab (2) is provided with hook, stem and/or cup-shaped fasteners (27) (these second tab fasteners may be identical or different to the first tab fasteners (25)) and the outer surface (3) at the first lateral side region (13) of the sleeve (1) has a structure or is provided with a structure (28) that is adapted to be engaged by the second tab fasteners (27). As can be appreciated from Figure 2, typically the first lateral side region (13) is provided with the relevant engagement structure (28) by laminating an appropriate layer of material onto the relevant region of the sleeve. In addition, the first lateral side region (13) can be easily trimmed along its outer edge. In the event, it is needed or desired to reduce the width or circumference of the compression device, in particular the sleeve thereof, the fastening tabs can be detached, an appropriate amount of the first lateral side region can be trimmed off, so as to achieve the needed or desired width/circumference. Thereafter the fastening tabs can be re-attached to the remaining portion of the first lateral side region, and the device applied. Generally the tabs are attached to the outer surface of the sleeve such that the tabs extend across the first lateral side edge, in particular so that their distal end portions are displaced from first lateral side edge.

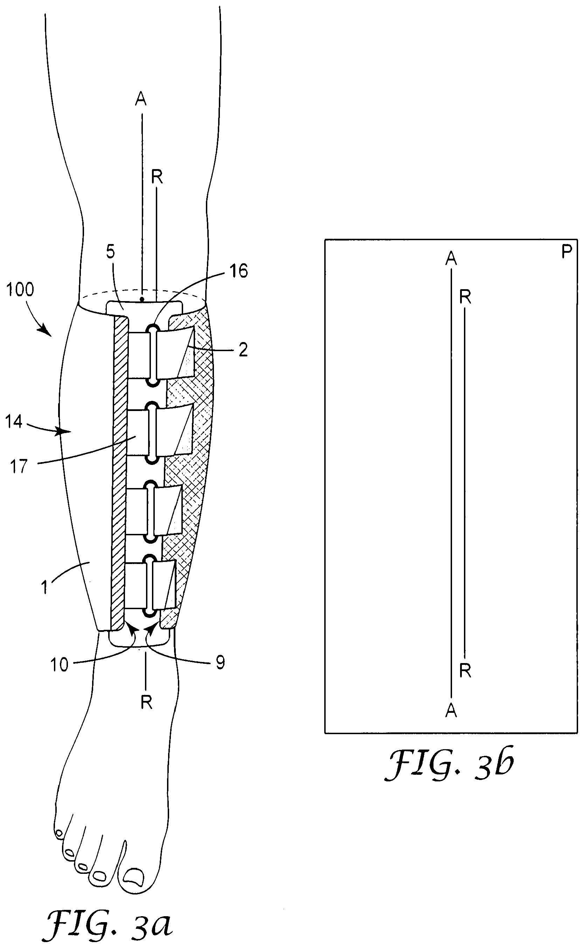

Referring to Figure 3a showing a perspective, front view of the exemplary compression device (100) depicted in Figures 1 and 2, in use on the lower leg of a user, it can be recognized that the fastening tabs (2) and rings (16) are configured and arranged such that, in use, the fastening tabs are passed through the rings, turned back on themselves such that the first lateral side edge (9) of the sleeve (1) is drawn towards the rings and finally fastened so that the sleeve is tightened and restrained about the limb of the user to provide compression. Once positioned onto the limb of the user, the compression device (100) advantageously encircles the relevant portion of the limb and, in this exemplary embodiment the sleeve (1) and in particular the central region (14) of the sleeve will encircle most of the limb.

For compression devices suitable for use with the lower leg of the user, favorably the sleeve is configured and arranged such that in use the rings, or for those embodiments having eyelets, the eyelets, will generally be positioned towards the front, in particular so that they extends generally along the tibia. Accordingly for such embodiments the central region of the sleeve will typically be positioned around the back and, at least on one of the sides of the lower leg, and thus accordingly next to the calf muscles.

Sleeves, in particular at least the central region thereof, favorably comprise a material that is suitable for use in applying compression. Such materials are known in the art. Favorably such materials have low flexural rigidity and are stretchable, so that they can readily adapt to the shape of the limb shape, while at the same time not being too easily stretched under tension, so that the desired provision of compression onto the limb can be achieved. More favorably at least the central region of the sleeve (even more favorably at least the central and the first lateral edge regions of the sleeve, most favorably the central region as well as the first and second lateral regions of the sleeve) comprises a short-stretch material having elasticity in at least the transverse direction in the sleeve and a maximum stretch greater than 0% up to 60% in at least the transverse direction of the sleeve under a load of 10 N per cm width (e.g. as measured in accordance with DIN 61632:2009). For comfort in wearing, such materials are desirably breathable. As indicated above, for embodiments including short-stretch compression materials, favorably the product of the modulus of elasticity (in the transverse direction) of the material of the loop (e.g. the outer-layer-material in the two-layer expandable strap portion/gusset embodiments) times the thickness of the material of the loop is favorably at least 90% of the product of the modulus of elasticity (in the transverse direction) of the short-stretch material times the thickness of the short-stretch material, in particular the product of the modulus of elasticity of the material of the loop (e.g. the outer-layer-material in the two-layer expandable strap portion/gusset embodiments) times the thickness of the material of the loop is equal to or greater than the product of the modulus of elasticity of said short-stretch material times the thickness of the short-stretch material.

Compression devices, in particular sleeves, may be provided with one or more stiffeners to facilitate maintenance of sleeve shape, in particular to minimize any tendency towards vertical collapsing or slipping-down of the sleeve, stiffeners may be provided e.g. in the form of wires, bars, grids, or pads having limited width relative to the transverse direction of the sleeve. In the exemplary embodiment depicted in Figures 1 and 2, for example, an elongate stiffener (42) that extends lengthwise between the upper and lower edges of the sleeve is provided in the second lateral side region (15) adjacent to the second lateral edge (10). Stiffeners may be made of e.g. metal or thermoplastic materials including thermoformable thermoplastic materials (such as polypropylene, polyamide, polyester (e.g. 3M Scotchcast Thermoplastic Material 72362)). For stiffeners having a width greater than five millimeters, it may be favorable to provide them with perforations to allow for breathability. For design and/or fixing purposes, stiffeners may be provided within a fabric pocket which is subsequently attached to the appropriate part(s) of the sleeve or alternatively stiffeners may be positioned on the surface of the appropriate part(s) of the sleeve, which are then covered completely with a sheet of fabric that is sewn or laminated onto the respective part(s) of the sleeve.

Returning to exemplary embodiment depicted in Figures 1 and 2 and referring to Figures 3a, it can be recognized that when the exemplary compression device (100) is in use on the limb of the user, the sleeve is disposed about a central axis (A) and the plurality of rings extends along a first axis (R). Making reference to Figure 3b it can be seen that relative to a projection of the first axis (R) onto said plane (P) containing the central axis (A), the first axis (R) is in parallel or essentially parallel alignment relative to the central axis. Generally it is favorable that the first axis (R) is either in parallel alignment relative to the central axis or nearly parallel alignment relative to the central axis (i.e. the first axis (R) may be inclined forming an acute angle of no more than 5° relative to the central axis). It is to be appreciated that when the compression device is in use on the limb of the user, it is possible that the series of rings may not extend along a perfectly straight axis, i.e. its projection may be curved due to tension and particular leg geometry of the user, and in such cases the relevant axis along which the series of rings extends may be defined as being the axis resulting from a best linear fit (linear regression) to the projected curve.

Comparing the compression device in use as depicted in Figure 3a to the same compression device not in use as depicted Figures 1 and 2, it can be seen in Figure 3a, loops on the ring-straps (17) have flattened out. Moreover in applying the exemplary compression device (100) depicted in Figures 1 and 2, the sleeve is positioned about the limb of the user, each of fastening tabs (2) is threaded through its opposing ring (16), turned back on itself and pulled such that the first lateral side edge (9) of the sleeve is drawn towards the rings so that the sleeve (1) is tightened about the limb of the user, wherein each tab is pulled until the loop in the expandable portion of the opposing ring strap fully flattens out. Once the loops disappear i.e. are no longer visible because they flattened out, the fastening tabs are fastened so that the sleeve (and correspondingly the compression device) is restrained about the limb of the user.

As it can be appreciated from the exemplary embodiment shown in Figures 1 and 2, it is favorable to configure and arrange the sleeve and fastening tabs, such that in use, when the fastening tabs are passed through the eyelets or rings, as applicable, turned back and fastened onto themselves, the first lateral edge is drawn towards the second lateral edge of the sleeve, but the two lateral edges of the sleeve do not overlap. Also as it can be appreciated from the exemplary embodiment shown in Figures 1 and 2, compression devices may favorably further comprise a tongue. The tongue is desirably configured and arranged relative to the sleeve such that, in use, the tongue is generally centrally positioned adjacent to and extends along the first and second lateral edges of the sleeve, so that the tongue is located between the user and an opening defined between the first and second lateral edges of the sleeve and so that the tongue underlies the rings.

Returning to the exemplary embodiment depicted in Figures 1 and 2, it can be seen that a tongue (5) is provided. The tongue, in particular a lateral edge portion thereof, is affixed to the inner surface (4) at the second lateral edge region (15) of the sleeve so that the tongue extends beyond the second lateral edge (10) and underlies the rings (16). Referring to Figure 3a, it can be recognized that the sleeve (1) and fastening tabs (2) of the exemplary compression device (100), are configured and arranged, such that in use, when the fastening tabs are passed through the eyelets or rings, as applicable, turned back and fastened onto themselves, the first lateral edge (9) is drawn towards the second lateral edge (10) of the sleeve, but the two lateral edges of the sleeve do not overlap. In addition it can be seen in Figure 3a, that when the compression device (100) is in use on the limb of the user, the tongue (5) is generally centrally positioned adjacent to and extends along the first and second lateral edges (9,10) of the sleeve (1), so that the tongue is located between the user and an opening defined between the first and second lateral edges of the sleeve.

For compression devices including a tongue, the tongue may favorably include either a single stiffener extending substantially across its width and length or one or more elongate stiffeners extending lengthwise. As mentioned above, stiffeners may be made of e.g. metal or thermoplastic materials including thermoformable thermoplastic materials (such as polypropylene, polyamide, polyester (e.g. 3M Scotchcast Thermoplastic Material 72362)). For stiffeners having a width greater than five millimeters, it may be favorable to provide them with perforations to allow for breathability. For design and/or fixing purposes, stiffeners may be provided within a fabric pocket which is subsequently attached to the appropriate part(s) of the sleeve or alternatively stiffeners may be positioned on the surface of the appropriate part(s) of the tongue, which are then covered completely with a sheet of fabric that is sewn or laminated onto the respective part(s) of the tongue. Alternatively or in addition, the tongue may comprise foam, in particular memory foam, more particular high density memory foam. High density memory foams are memory foams that have a density of at least 65 kg/m3, in particular at least 70 kg/m³, more particularly at least 85 kg/m³, most particularly at least 105 kg/m³. Examples of suitable memory foams, include high density memory foams available from Filtrona Porous Technologies marketed under the trade designations SRF EP2, Argus, Argus Soft, and Argus Supersoft. Favorably the tongue comprises a layer of foam, in particular a layer of foam having a thickness from 0.5 mm to 10 mm, inclusive, more particular a layer of foam having a thickness from 2 mm to 6 mm, inclusive.

Returning to the exemplary embodiment depicted in Figures 1 and 2, it can be seen, in particular in Figure 2, that the tongue (5) includes two elongate stiffeners (42) extending lengthwise. Desirably the two elongate stiffeners are positioned such that in use when the loop is just flattened out, the rings will be located between the two elongate stiffeners. The stiffeners are located between two layers, an inner layer (6) of foam and an upper layer (36) made of an appropriate covering material.

Although not specifically shown in the exemplary embodiments depicted herein, compression devices described herein may be configured to include other structural elements, for example a foot portion extending from the sleeve, in particular extending from an appropriate portion of the lower edge of the sleeve. Such a foot portion may be configured and arranged in the form of a stir-up or alternatively such as foot portion may be configured to provide a more extensive covering of the foot. Moreover the sleeve and such a foot portion may be configured and arranged so as to provide a boot-like compression device, either closed or opened toed and/or either closed or opened heeled. Such a foot part may be provided integrally with the sleeve or alternatively as a separate component that can be attached to the sleeve by an appropriate fastening means, such as buttons, mechanical fasteners and the like. Compression devices may also include bladders or gel inserts to facilitate modification of circumferential size. In this regard, sleeves, for example, could be provided with double walls or interior pockets for such inserts so that such insert(s) may be inserted and/or removed as needed or desired.

As indicated above, compression devices including an expandable strap portion with a loop-indicating configuration, it is favorable to configure the device such that when said portions of the straps are their non-expanded state, the rings are positioned along the second lateral edge of the sleeve either adjacent to or spaced apart from said edge and away from the second lateral edge portion, in particular wherein each ring has a lateral edge near the second lateral edge of the sleeve, said lateral edge of the ring is either positioned adjacent to second lateral edge of the sleeve or spaced apart from the second lateral edge at a distance of at most 4 cm, in particular at most 3 cm. Such embodiments favorably include a tongue as described above.

Alternatively, for compression devices including an expandable strap portion with a loop-indicating configuration, it may be desirable to configure the device such that when said portions of the straps are their non-expanded state, the rings are spaced apart from the second lateral edge towards the central region, in particular wherein each ring has a lateral edge distant to the central portion of the sleeve, said lateral edge of the ring is spaced apart from the second lateral edge at a distance between 3 and 25 cm (inclusive the end points).

The exemplary embodiment depicted in Figures 4 and 5 is an example of such an alternative compression device (100). This exemplary compression device (100) comprises like the exemplary embodiment depicted in Figures 1 and 2, a sleeve (1) for substantially covering a portion of the limb of a user where the second lateral edge region (15) of the sleeve is provided with a plurality of rings (16) in series between the upper and lower edges (7, 8) of the sleeve. However in this embodiment, when the expandable portions (21) of the straps (17) are in their non-expanded state, the rings (16) are spaced apart from the second lateral edge (10) towards the central region (14), in particular wherein each ring has a lateral edge (32) distant to the central portion of the sleeve, and this lateral edge of the ring is spaced apart from the second lateral edge (for example at a distance from 3 cm up to and including 25 cm). So in use when the compression device (100) is applied onto the limb of the user, when the first lateral edge (9) is drawn towards the rings (16), the first lateral edge will be pulled over the second lateral edge (10) so that a portion of the second lateral edge region (15) adjacent to the second lateral edge will form an under-winding. The exemplary compression device (100) comprises a series of fastening tabs (2) that are releasably attachable to the first lateral edge region (13), wherein the tabs and first lateral edge region are configured the same as that described in connection with the exemplary embodiment depicted in Figures 1 and 2. The present exemplary compression device differs from first exemplary embodiment in that the rings (16) together with their straps (17) are releasably attachable to the sleeve (1), in particular to the second lateral edge region (15). Moreover in addition to the expandable, loop-comprising strap portion (21), the strap (17) includes a proximal end portion (31), wherein the inner major surface (34) (i.e. that surface facing the outer surface of the sleeve and opposite to the outwardly facing surface of the strap which includes the loop (20)) at the proximal end portion of the strap is provided with hook, stem and/or cup-shaped fasteners (strap fasteners) (37). Also, the outer surface (3) at the second lateral edge region (15) of the sleeve has a structure or is provided with a structure (38) that is adapted to be engaged by strap fasteners (37). Accordingly the inner surface (34) at the proximal end portion (31) of the strap may be then releasably attached to the outer surface (3) at second lateral edge region (15) of sleeve (1). As can be appreciated from the exemplary embodiment, it may be advantageous that the first lateral edge region (13), the central region (14) and second lateral edge region (15) are configured the same, i.e. the outer surface (3) at each region has a structure or is provided with a structure (38) that is adapted to be engaged by the second tab fasteners (27) and strap fasteners (37). It will be appreciated that the second tab fasteners and strap fasteners may be identical. Favorably both the first and second lateral edge regions (13, 15) of the sleeve are configured such that they are trimmable. Such a configuration allows for an advantageously high degree of freedom in regard to width and/or circumference adjustment. This can be especially useful for those users having an unusual and/or non-standard limb width/circumference where a custom sizing normally would be required necessary. Moreover, the sleeve material could be advantageously provided on a roll, where a length of sleeve material (the length corresponding to targeted, desired/needed width or circumference) could be cut from the roll and thereafter the fastening tabs and the rings with their straps (each of which includes the described proximal end portion and expandable, loop-comprising portion) could be then releasably attached to the outer surface of the sleeve to provide a compression device. While in the illustrated exemplary embodiment the fastening tabs and the rings with their straps are each, individually releasably attachable to the sleeve, in alternative embodiments the series of fastening tabs could be configured and arranged as a single, integral component that is releasably attachable to the sleeve. Similarly the series of rings with their straps could be configured and arranged as a single, integral component that is releasably attachable to the sleeve. Such integral tab or ring-strap components could in addition comprise one or more stiffeners.

Generally, for compression devices including rings and straps having an expandable portion with a loop-indicating configuration as described above, desirably said expandable portion of the strap has in its non-expanded state a width relative to the transverse direction of the sleeve of at least 0.1 cm, in particular at least 0.5 cm. Desirably the expandable portion of the strap has in its non-expanded state a width relative to the transverse direction of the sleeve of at most 4 cm, more desirably at most 3 cm. Favorably the expandable portion of the strap has in its expanded state at the point where the loop just fully flattens out a width relative to the transverse direction of the sleeve of at least 1 cm. Favorably the expandable portion of the strap has in its expanded state at the point where the loop just fully flattens out a width relative to the transverse direction of the sleeve of at most 8 cm, more favorably at most 6 cm.

Generally, for compression devices including rings and straps having an expandable portion with a loop-indicating configuration as described above, favorably each strap has a height relative to the transverse direction of the sleeve of at least 1 cm, more favorably at least 2 cm, and most favorably at least 3 cm. Favorably each strap has a height relative to the transverse direction of the sleeve of at most 10 cm, more favorably at most 8 cm, and most favorably at most 6 cm. In addition or alternatively, desirably the plurality of rings or straps including the interstices between rings or straps, respectively, extends over a height corresponding to at least 70% of the height of the sleeve from the upper to lower edge. The plurality of rings or straps including the interstices between rings or straps, respectively, may extend over a height corresponding from 70% up to 100% and, possibly greater than 100% of the height of the sleeve from the upper to lower edge. Favorably the height of the interstices between rings or straps ranges from 0.1 mm to 7 cm, inclusive, more favorably from 0.3 mm to 3 cm, inclusive, and most favorably from 0.5 mm to 2 cm.

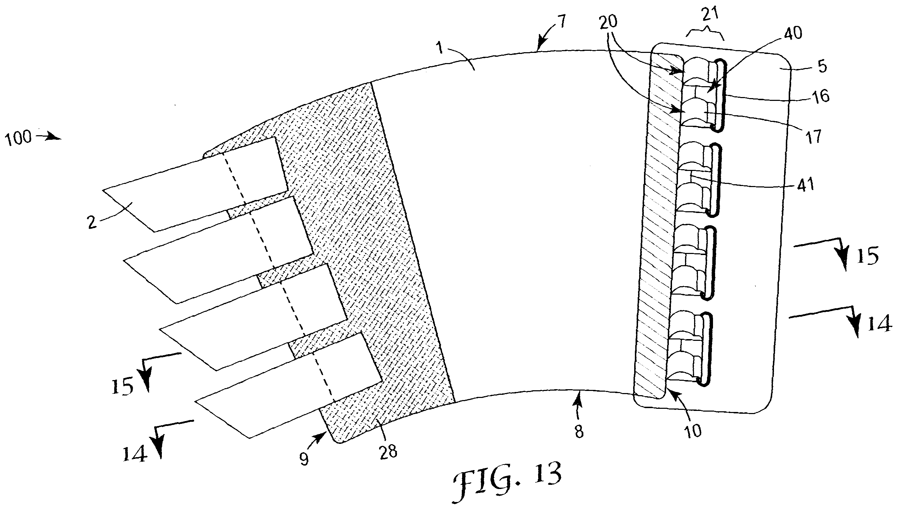

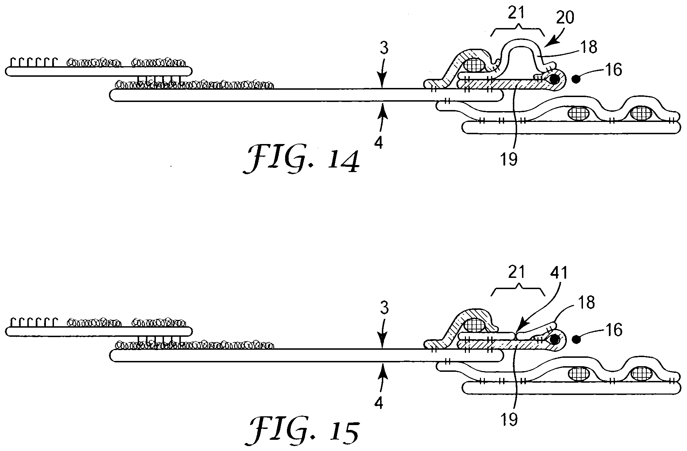

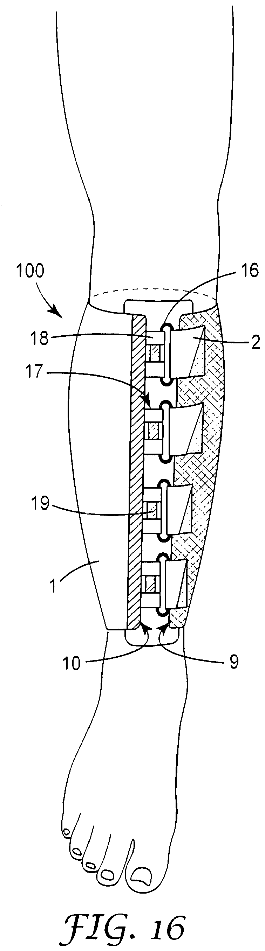

Figures 13 to 15 provide illustrations of an exemplary embodiment similar to that shown in Figures 1 and 2, except this embodiment is provided with a slit-region in the expandable strap portion of the straps. Moreover the exemplary compression device (100) shown in Figures 13 to 15 is identical to that shown in Figures 1 and 2 except for the configuration of the outer-layer and inner-layer-materials (18, 19, respectively) of the expandable portion (21) of each of the straps (17). Referring to Figure 13, it can be seen that the expandable portion of each strap includes two loops (20) and one slit region (40) alternatively (i.e. loop-slit region-loop) and in series lengthwise relative to said upper and lower edges (7,8, respectively) of the sleeve (1) (i.e. substantially along the longitudinal direction of the sleeve). Referring to Figure 13, it can be appreciated that the outer-layer (18) of the expandable portion in its non-expanded state shows a central /-like cut so that the outer layer material is essentially divided into three sections, which can be then configured and arranged in conjunction with the inner-layer as to provide the loops and the slit region. The configuration and arrangement of the outer and inner layer materials are best seen in Figures 14 and 15. The two outer sections of the outer layer are affixed to the inner layer such that two loops (20) of outer-layer-material (18) are provided above the inner layer (19) when the expandable strap portion (21) is in its non-expanded state (see e.g. Figure 14). Like the other exemplary embodiments described herein, these loops can flatten when the sleeve is used (i.e. applied) and under the provision of tension the expandable portion of the straps expands in the transverse direction. The central section of the outer layer material (18) is also positioned above the inner layer (19) and the outer-layer material includes an opening extending substantially lengthwise relative to said upper and lower edges of the sleeve. As can be appreciated from Figure 15, when the expandable strap portion (21) is in its non-expanded state, the opening has a form of a slit (41) and the outer-layer material (18) lies substantially flat with the lateral edges of the slit near to one another so the underlying inner layer material is covered. (In alternative embodiments the inner and outer layers can be configured and arranged such that the lateral edges of the slit overlap.) It will be appreciated excess outer-layer material from the middle of the central section is removed, e.g. cut out, to be sure that when the expanded portion is in its non-expanded state, the outer-layer-material lies substantially flat with the lateral edges of the slit adjacent to (or in alternative embodiments overlapping) one another, while the outer sections of the outer-layer material show loops of outer-layer material over the inner layer. Referring to Figure 16 showing a perspective, front view of the exemplary compression device (100) depicted in Figures 13 to 15, in use on the lower leg of a user, it can be recognized when the fastening tabs (2) are pulled through the rings (16) such that the first lateral side edge (9) of the sleeve (1) is drawn towards the rings and the second lateral side edge (10) of the sleeve so that the sleeve is tightened and restrained about the limb of the user to provide compression, the expandable portions of the strap expand in the transverse direction and in turn the lateral edges of the slit in the outer layer material (18) move apart exposing the underlying inner layer (19) while at the same time the loops (not visible in Figure 16) of the outer layer material flattening. Once the slit opens exposing the underlying inner layer and the loops fully flatten, the user can fastened the sleeve.

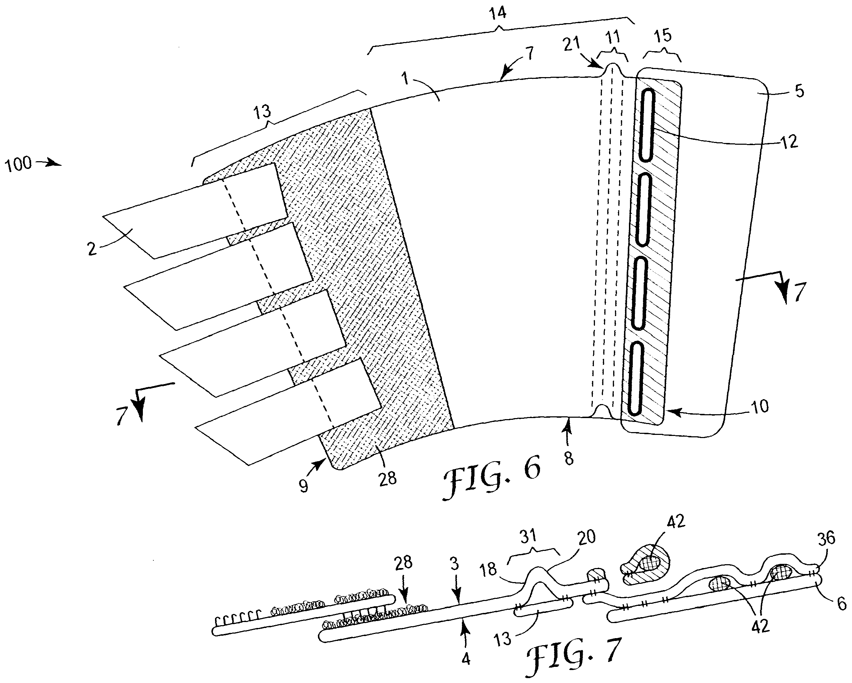

Figures 6 to 12 depict exemplary embodiments where the loop-indicating configuration is provided in an elongate, expandable gusset that extends substantially lengthwise between the upper and lower edges of the sleeve. Generally, for compression devices that include such a gusset, the gusset favorably extends a height corresponding to 70% up to 100% of the height of the sleeve from the upper to lower edge, more favorably the gusset extends from the upper to lower edges of the sleeve. Similar to the expandable portion of the strap described above, gussets comprise a material having elasticity in at least the transverse direction in the sleeve and are configured and arranged such that when the gusset is in its non-expanded state (e.g. when the compression device is not in use) there is to the exterior of the device a loop of material rising outwardly and when in use under the provision of tension in the transverse direction of the sleeve, the gusset expands in the transverse direction and the loop flattens (eventually disappearing).

Figure 6 shows a top view of the exterior of an exemplary embodiment of a compression device (100) for use in applying compression to a limb of a user including instead of rings, eyelets, while Figure 7 shows a cross-sectional view of this exemplary embodiment. The device comprises a sleeve (1) for substantially covering a portion of the limb of a user. The sleeve includes an outer surface (3), an inner surface (4), an upper edge (7), a lower edge (8) and two lateral side edges (9, 10). As in the other exemplary embodiments, in the transverse direction from the first lateral side edge (9) to the second lateral side edge (10) the sleeve comprises a first lateral side region (13), a central region (14) and a second lateral side region (15). The second lateral edge region of the sleeve is provided with either a plurality of eyelets (12) in series between the upper and lower edges of the sleeve. As can be appreciated from the Figures, in particular Figure 7, the second lateral edge region (15) also favorably includes a stiffener (42) that is located between the eyelets (12) and the second lateral edge (10). The sleeve also includes an elongate, expandable gusset (11) extending substantially lengthwise between the upper and lower edges (7, 8) of the sleeve, in particular the gusset extends from the upper to the lower edges of the sleeve. The gusset is expandable in at least the transverse direction of the sleeve and includes a loop (20) of material to the exterior and rising outwardly, which can be better seen in cross-sectional view shown in Figure 7. It can also be seen that the expandable gusset (11) favorably comprises two layers, an outer layer of material (18) and an inner layer of material (19), At least one of (desirably both) the inner-layer-material and outer-layer-material has (have) elasticity in at least the transverse direction. As can be appreciated from Figure 7, the outer layer of the gusset is integral with the adjacent-lying material of the sleeve with the inner layer of the gusset being a separate strip of material affixed to the inner surface of the sleeve, so as to provide a loop of outer-layer-material (i.e. the loop (20) of the gusset) above the inner layer. The loop (20) is visible as an elongate mound or hump. In use, when the device (100) is applied onto the limb of a user, tension will be provided and accordingly the expandable gusset (11) will expand in the transverse direction and the loop can and will flatten.

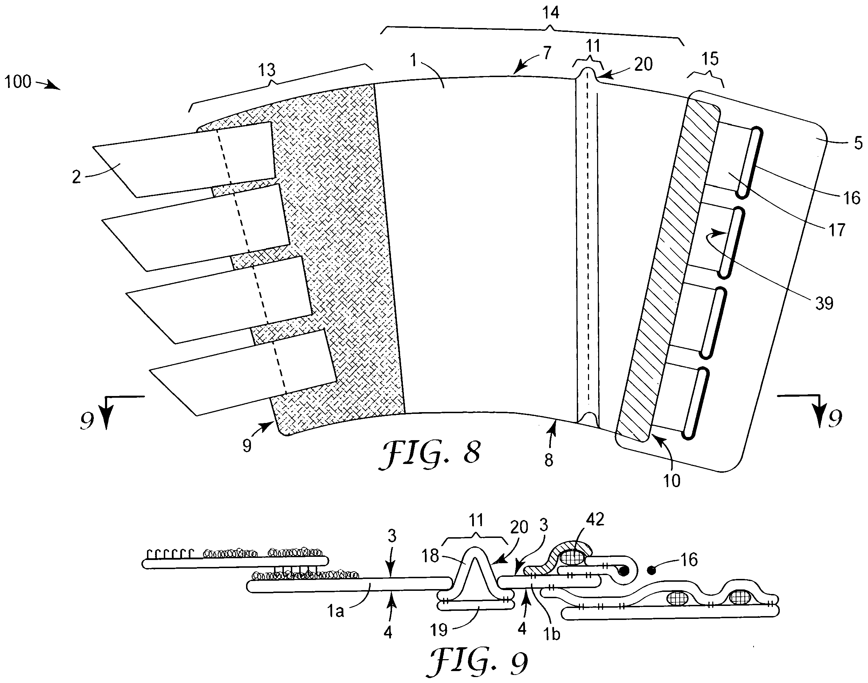

Gussets may be at least in part integral with adjacent lying sleeve material or alternatively gussets may be provided as inset into the sleeve (the exemplary embodiment depicted in Figures 8 and 9 is an example of the latter). For those embodiments where the expandable gussets includes two layers, as already indicated above, favorably the product of the modulus of elasticity of the inner-layer-material times the thickness of the inner-layer-material is less than the product of the modulus of elasticity of the outer-layer-material times the thickness of the outer-layer-material. More favorably, the product of the modulus of elasticity of the inner-layer-material times the thickness of the inner-layer-material is at least a factor of two times, more favorably at least a factor of four times, lower the product of the modulus of elasticity of the outer-layer-material times the thickness of the outer-layer-material. For those embodiments including a two layer gusset and where the gusset is at least in part integral with the adjacent lying sleeve material, favorably the loop material, i.e. the outer-layer-material, is integral with the adjacent-lying material of the sleeve, while then the inner layer of the gusset is a separate strip of material affixed to the inner surface of the sleeve, so as to provide a loop of outer-layer-material above the inner layer. For such embodiments which further favorably comprise short-stretch material in the relevant adjacent region(s) of the sleeve, it will be appreciated that the modulus of elasticity and thickness of the loop material, i.e. the outer-layer-material in the two-layer expandable gusset, will be normally equal to the modulus of elasticity and thickness, respectively, of said short-stretch material. Otherwise as already indicated above, for embodiments including a short-stretch material in the relevant region(s) of the sleeve as described above, favorably the product of the modulus of elasticity of the material of the loop (e.g. the outer-layer-material in two-layer gusset embodiments) times the thickness of the material of the loop is favorably at least 90% of the product of the modulus of elasticity of the short-stretch material times the thickness of the short-stretch material, in particular the product of the modulus of elasticity of the material of the loop (e.g. the outer-layer-material in two-layer expandable gusset embodiments) times the thickness of the material of the loop is equal to or greater than the product of the modulus of elasticity of said short-stretch material times the thickness of the short-stretch material.

The exemplary compression device in Figures 6 and 7 further comprises a plurality of strip-shaped mechanical fastening tabs (2), one fastening tab for each eyelet. The fastening tabs (2) as well as the first lateral side portion (13) is favorably configured as described above. The exemplary device also includes a tongue (5) desirably affixed to the inner surface (4) at the second lateral edge region (15) so that the tongue underlies eyelets and extends outwardly from the second lateral edge (10). The tongue is favorably configured as described above, and as can be seen in Figure 7, the tongue of the exemplary embodiments includes two elongate stiffeners (42) extending lengthwise located between an outer layer (36) and inner layer (6) of material, the latter of which is favorably made of foam.

Although not shown in an illustration, it will be recognized that when the exemplary compression device depicted in Figures 6 and 7 is put to use covering the limb of a user (e.g. the lower leg including the calf of a user), the fastening tabs (2) will be passed through the eyelets (12), turned back and fastened onto themselves. The first lateral edge (9) will be thus drawn towards eyelets (12) and accordingly towards the second lateral edge (10) of the sleeve (1). Favorably the two lateral edges of the sleeve will not overlap, but will define an opening behind which the tongue (5) will be centrally positioned. Moreover in applying the compression device depicted in Figures 6 and 7, the sleeve (1) is positioned about the limb of the user, each of fastening tabs (2) is threaded through its opposing eyelet (12), turned back on itself and pulled such the first lateral side edge (9) of the sleeve is drawn towards the eyelets (12) so that the sleeve is tightened about the limb of the user, wherein the tab is pulled until the loop of the expandable gusset fully flattens out. Once the loop disappears, the fastening tabs are fastened so that the sleeve (and correspondingly the compression device) is restrained about the limb of the user.

Gussets desirably have in their non-expanded state a width relative to the transverse direction of the sleeve of at least 0.1 cm, more desirably at least 0.5 cm. Gussets desirably have in their non-expanded state a width relative to the transverse direction of the sleeve of at most 4cm, more desirably at most 3 cm. Favorably gussets have in their expanded state at the point where the loop just fully flattens out a width relative to the transverse direction of the sleeve of at least 1 cm. Favorably gussets have in their expanded state at the point where the loop just fully flattens out a width relative to the transverse direction of the sleeve is at most 8 cm, in particular at most 6 cm.

Gussets may be provided anywhere in the sleeve between the fastening tabs and eyelets (or rings, for those embodiments including rings instead of eyelets). For ease in viewing loop-indicating configuration of the gusset while the device is being applied, suitably gussets may be provided in the central portion of the sleeve or in the second lateral side portion, more suitably in the central portion of the sleeve near the eyelets (or rings, if applicable) or in the second lateral side portion near or adjacent to the eyelets (or rings, if applicable) towards the central portion.

Returning to the exemplary embodiment depicted in Figures 6 and 7, it can be seen that the gusset is in the central portion (14) of the sleeve with the gusset being positioned near the eyelets (12) and thus distant to the fastening tabs (2) attached to the first lateral edge region (13) of the sleeve. Although not shown in an illustration, it will be appreciated that when this exemplary compression device (100) is in use on the limb of the user, the sleeve (1) will be disposed about a central axis (A), said central axis lying in a plane (P), and the gusset will extend along a second axis (G), wherein relative to a projection of this second axis (G) onto said plane (P) containing the central axis (A), the second axis (G) will be in parallel or essentially parallel alignment with the central axis (A). In alternative embodiments, gussets can be configured and arranged such that the aforesaid second axis (G) is inclined forming an acute angle (β) up to 25° inclusive relative to the central axis.

The exemplary compression device shown in Figures 8 and 9 is an example of an embodiment where the expandable gusset (11) is arranged such that when the compression device (100) is in use on the limb of the user, the gusset extends along a second axis (G), wherein relative to a projection of the second axis (G) onto said plane (P) containing the central axis (A), the second axis (G) is inclined forming an acute angle (β) relative to the central axis (A). In particular, the exemplary compression device shown in Figures 8 and 9 includes a sleeve (1) having instead of eyelets, a plurality of rings (16) in series between the upper and lower edges (7, 8) of the sleeve. Each ring is favorably fixedly attached by a strap (17) extending between the sleeve (1) and the ring (16). The rings of this exemplary device are positioned along the second lateral edge (10) of the sleeve and spaced apart from said edge and away from the second lateral edge region (15), in particular the lateral edge (39) of each ring which is near the second lateral edge (10) of the sleeve is spaced apart from the second lateral edge of the sleeve. Favorably the spacing corresponds to a distance of at most 4 cm, more favorably at most 3 cm. Alternatively, rings may be positioned adjacent to the second lateral edge (but still away from the second lateral edge region (15)), in particular, the lateral edge (39) of each ring which is near the second lateral edge (10) of the sleeve may be positioned adjacent to the second lateral edge of the sleeve. From Figure 9, it can be seen in this exemplary embodiment that the ring-straps are attached to the second lateral edge region (15) of the sleeve and the region (15) of the sleeve is provided with a stiffener (42). The exemplary embodiment includes fastening tabs (2) and a tongue (5), both elements configured and arranged as previously described above. In regard to the expandable gusset (11), it can be recognized from Figure 9 that in this exemplary embodiment the gusset is provided as an inset provided in the sleeve, in particular in the central region (14) thereof. Moreover referring to the illustration of Figure 9, it can be seen that the sleeve is made of two individual parts (1a and 1b) and that the gusset (11) comprises an outer layer of material (18) and inner layer of material (19), wherein the inner layer of material is affixed to the inner surface of the outer layer of material and configured and arranged so as to provide a loop (20) of outer-layer-material above the inner layer, and wherein the gusset is then affixed to inner surface (4) of the each of two sleeve parts (1a, 1b), thus bridging the two sleeve-parts to provide a complete sleeve with the loop (20) facing outwardly.

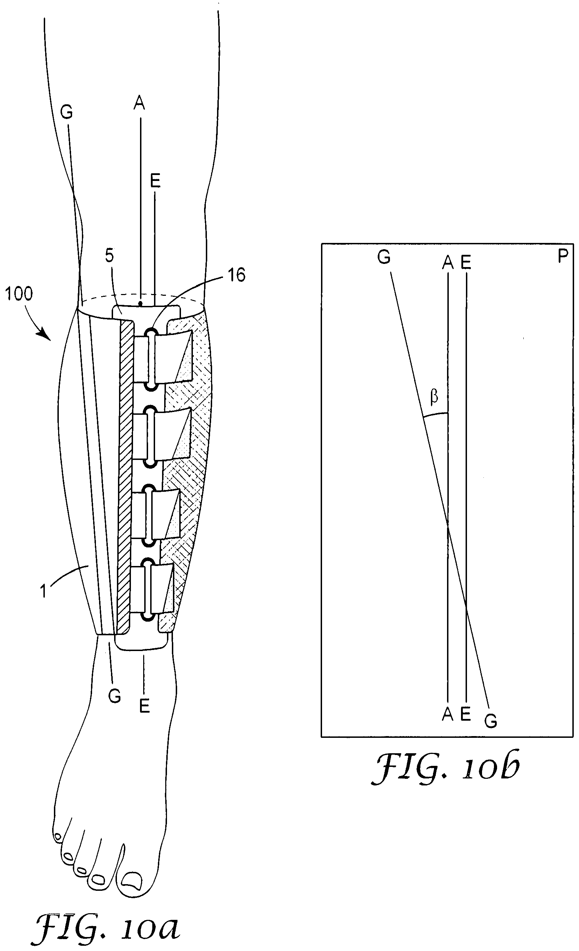

Referring to the illustration of Figure 8, it can be seen that the gusset of this exemplary embodiment is inclined relative to e.g. the second lateral edge (10). Moreover, reference is made to Figure 10a showing a perspective, front view of the exemplary compression device (100) depicted in Figures 8 and 9, in use on the lower leg of a user, it can be recognized that the sleeve (1) is disposed about a central axis (A) and the gusset (11) extends lengthwise along a second axis (G). Making reference to Figure 10b showing a projection of this axis (G) on the plane (P) containing the central axis (A) it can be appreciated that relative to a projection of the second axis (G) onto said plane (P) containing the central axis (A), the second axis (G) is inclined forming an acute angle (β) of about 12°relative to the central axis. Returning to Figure 10a, one can also see that when the compression device (100) is in use, the gusset (11) will expand in the transverse direction and the loop will flatten. As suggested in Figure 10a, when the device is properly applied the gusset will be expanded such that the loop is fully flattened out, i.e. it disappears.

As can be appreciated from Figure 10b, generally for compression devices comprising expandable gussets as described herein, when such devices are in use on the limb of the user, the sleeve is disposed about a central axis (A), said central axis lying in a plane (P), and the plurality of eyelets or rings, as applicable, favorably extends along a third axis (E), wherein relative to a projection of the third axis (E) onto said plane (P) containing the central axis (A), the third axis is either in parallel alignment or nearly parallel alignment relative to the central axis (i.e. the third axis (E) may inclined forming an acute angle of no more than 5° relative to the central axis).

Generally for compression devices comprising expandable gussets as described herein, favorably the plurality of eyelets or rings, as applicable, including the interstices between eyelets or rings, respectively extends over a height corresponding to at least 70% of the height of the sleeve from the upper to lower edge. Desirably the height of the interstices between eyelets and rings, as applicable, ranges from 0.1 mm to 7 cm, inclusive, in particular from 0.3 mm to 3 cm, inclusive, and more particular from 0.5 mm to 2 cm. For those embodiments including rings and straps favorably each strap has a height relative to the transverse direction of the sleeve of at least 1 cm, more favorably at least 2 cm, most favorably at least 3 cm. Desirably each strap has a height relative to the transverse direction of the sleeve of at most 10 cm, more desirably at most 8 cm, most desirably at most 6 cm. It is favorable that the height of the interstices between straps ranges from 0.1 mm to 7 cm, inclusive, in particular from 0.3 mm to 3 cm, inclusive, and more particular from 0.5 mm to 2 cm.

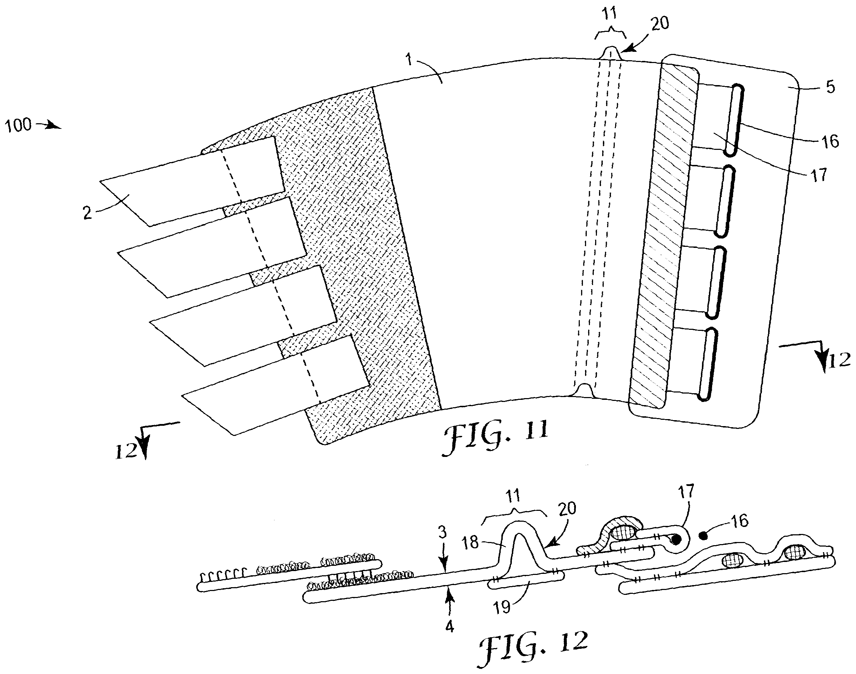

The exemplary compression device shown in Figures 11 and 12 is a variant of the exemplary embodiment shown in Figures 8 and 9 and differing in two aspects. The first being that the gusset (11) is not provided an inset, but rather the outer layer (18) of the gusset (11) is integral with the adjacent-lying material of the sleeve (1) with the inner layer (19) of the gusset being a separate strip of material affixed to the inner surface (4) of the sleeve. Secondly the gusset (11) extends lengthwise such that when the compression device (100) is in use on the limb of the user, the gusset extends along a second axis (G), wherein relative to a projection of the second axis onto said plane (P) containing the central axis (A), the second axis (G) is parallel or essentially parallel to the central axis.

As will be appreciated from the exemplary embodiments described herein, to facilitate application and an overall smooth fit of the device, eyelets and rings, as applicable, are favorably configured and/or selected, such that the opening of the eyelet or ring has a height relative to the transverse direction of the sleeve which is greater than the height relative to the transverse direction of the sleeve of the fastening tab. And for those embodiments including rings and straps, favorably the rings are configured and/or selected, such that the opening of the eyelet or ring has a height relative to the transverse direction of the sleeve which is greater than the height relative to the transverse direction of the sleeve of the strap. In addition or alternatively, eyelets and rings, as applicable, are desirably rectangular or substantially rectangular in form; or oval or substantially oval in form (e.g. narrow or elongate oval, canoe-form, elongate teardrop); or a elongate or narrow D-shape in form.

Fastening tabs desirably have a height relative to the transverse direction of the sleeve of at least 1 cm, more favorably as least 2 cm. more desirably at least 3 cm. Fastening tabs desirably have a height relative to the transverse direction of the sleeve of at most 10 cm, more desirably at most 8 cm, most desirably at most 6 cm. Fastening tabs desirably have a width relative to the transverse direction of the sleeve of at least 6 cm. Fastening tabs desirable have a width relative to the transverse direction of the sleeve is at most 25 cm.

Straps desirably have a height relative to the transverse direction of the sleeve of at least 1 cm, more desirably at least 2 cm, most desirably at least 3 cm. Desirably straps have a height relative to the transverse direction of the sleeve of at most 10 cm, more favorably at most 8 cm, most favorably at most 6 cm.

As previously indicated methods of applying a compression device described herein onto the limb on a user, generally comprises the steps of:
a) positioning the sleeve about the limb of the user;
b) passing each fastening tab through its opposing eyelet or ring, as applicable;
c) turning each fastening tab back on itself;
d) pulling the fastening tabs so that the first lateral side edge of the sleeve is drawn towards the eyelets or rings, as applicable;
e) fastening the fastening tabs once the fastening tabs have been pulled and the expandable strap-portion or gusset, as applicable, have expanded to an extent, such that the loop material is fully flattened out (in particular desirably the fastening is performed just when the loop material has fully flattened out (and accordingly disappeared)).

And for those embodiments including a slit-region, one would favorably fastening the fastening tabs in step e) once the fastening tabs have been pulled and the expandable strap-portion or gusset, as applicable, have expanded to an extent, such that the lateral edges of the slit have moved apart exposing the underlying inner layer and the loop material is fully flattened out. For embodiments including a slit-region, it will be appreciated that after proper application of the device and in the event the tension decreases (due to decreasing volume of the leg (e.g. as a result of edema shrinkage) or for some other reason, such as material fatigue) the lateral edges of the slit will move towards one another, and as soon as the underlying inner layer and/or, if applicable, indicia provided on the underlying inner layer are no longer visible or, if applicable, only partially visible, this will provide the user and/or care-giver an indication that the device needs to be refastened and re-tightened. This indication will be reinforced by an observation of the loop material starting to re-form its loop, again due the tension decreasing. But even if the user and/or care-giver is unsure whether the loop material is starting to bump up or not, as soon as the underlying inner layer and/or indicia thereon (if applicable) in the slit-region are no longer visible or, only partially visible (if applicable), one has an indication that it is time to re-tighten the compression device.

## Claims

1. A compression device for applying compression to a limb of a user comprising a sleeve for substantially covering a portion of the limb of a user, wherein the sleeve has an outer surface, an inner surface, an upper edge, a lower edge and two lateral side edges, wherein in the transverse direction from the first lateral side edge to the second lateral side edge the sleeve comprises a first lateral side region, a central region and a second lateral side region, wherein the second lateral edge region of the sleeve is provided with a plurality of rings in series between the upper and lower edges of the sleeve, each ring being releasably or fixedly attached by a strap extending between the sleeve and the ring in substantially the transverse direction of the sleeve, at least a portion of said strap being expandable in at least the transverse direction, wherein said expandable portion comprises a material having elasticity in at least the transverse direction and is configured and arranged, such that when the expandable portion is in its non-expanded state there is exteriorly a loop of material rising outwardly and when, in use under the provision of tension in the transverse direction of the sleeve, the expandable portion expands in the transverse direction and the loop flattens;
wherein the device further comprises a plurality of strip-shaped mechanical fastening tabs, wherein a single tab is provided for each ring, each tab comprising a proximal end portion and a distal end portion being connected by an inner tab portion, wherein said proximal end portion is releasably or fixedly attached to the first lateral edge region of sleeve such that the tab is located opposite to a ring and extends in substantially the transverse direction of the sleeve, with its distal end portion positioned away from the central portion of the sleeve, wherein each tab has a first major surface located towards the outer surface of the sleeve and a second major surface located away from the outer surface of the sleeve, wherein the second major surface at the distal end portion of the tab comprises one part of a mechanical fastening system and said second major surface at the proximal end portion of the tab comprise the complementary part of the mechanical fastening system; and wherein the tabs and rings are configured and arranged such that, in use, the tabs are passed through the rings, turned back on themselves such that the first lateral side edge of the sleeve is drawn towards the rings and then fastened so that the sleeve is tightened and restrained about the limb of the user.

2. A compression device according to any one of the preceding claims, wherein the expandable portion of the strap comprises two layers, an outer layer of material and an inner layer of material, the product of the modulus of elasticity of the inner-layer-material times the thickness of the inner-layer-material being less than the product of the modulus of elasticity of the outer-layer-material times the thickness of the outer-layer-material, wherein outer-layer-material is said loop material and wherein the inner layer of material is affixed to the outer layer of material, so as to provide a loop of outer-layer-material above the inner layer when the expandable strap portion is in its non-expanded state, which, in use under the provision of tension and accordingly expansion of expandable portion of the strap in the transverse direction, can flatten.

3. A compression device according to any one of claims 1 to 2, wherein the expandable portion of the strap comprises two layers, an outer layer of material and an inner layer of material, the product of the modulus of elasticity of the inner-layer-material times the thickness of the inner-layer-material being less than the product of the modulus of elasticity of the outer-layer-material times the thickness of the outer-layer-material, wherein the inner layer of material and outer layer of material are configured and arranged, so as to provide:
at least one loop of outer-layer-material above the inner layer when the expandable strap portion is in its non-expanded state which, in use under the provision of tension and accordingly expansion of expandable portion of the strap in the transverse direction, can flatten; and
at least one region of outer-layer material above the inner layer, where the outer-layer material includes a slit extending substantially lengthwise relative to said upper and lower edges of the sleeve (slit-region), so that when the expandable strap portion is in its non-expanded state, the outer-layer material in said at least one slit-region lies substantially flat with the lateral edges of said slit near to or overlapping one another and in use, under the provision of tension and accordingly expansion of expandable portion of the strap in the transverse direction, the lateral edges of the said slit move apart exposing the underlying inner layer.

4. A compression device according to claim 3, wherein the inner layer is provided with indicia that become visible when the lateral edges of the slit move apart and/or the inner layer is provided with a color that is different to the color of the outer-layer.

5. A compression device according to claim 1, wherein, when said portions of the straps are in their non-expanded state, the rings are spaced apart from the second lateral edge towards the central region, in particular wherein each ring has a lateral edge distant to the central portion of the sleeve, said lateral edge of the ring is spaced apart from the second lateral edge at a distance between 3 and 25 cm, inclusive the endpoints, and wherein the rings are releasably attached to the outer surface at the second lateral edge region, wherein each strap further comprises a proximal end portion, wherein the inner surface at the proximal end portion of the strap is provided with hook, stem and/or cup-shaped fasteners (strap-fasteners) and wherein the outer surface at the second lateral edge region of the sleeve has a structure or is provided with a structure that is adapted to be engaged by said strap-fasteners.

6. A compression device for applying compression to a limb of a user comprising a sleeve for substantially covering a portion of the limb of a user, wherein the sleeve has an outer surface, an inner surface, an upper edge, a lower edge and two lateral side edges, wherein in the transverse direction from the first lateral side edge to the second lateral side edge the sleeve comprises a first lateral side region, a central region and a second lateral side region, wherein the second lateral edge region of the sleeve is provided with either a plurality of eyelets or a plurality of rings in series between the upper and lower edges of the sleeve, wherein the sleeve includes an elongate, expandable gusset extending substantially lengthwise between the upper and lower edges of the sleeve, said gusset being expandable in at least the transverse direction of the sleeve, wherein said expandable gusset comprises a material having elasticity in at least the transverse direction and is configured and arranged, such that when the expandable gusset is in its non-expanded state there is exteriorly a loop of material rising outwardly, and when, in use under the provision of tension in the transverse direction of the sleeve, the expandable gusset expands in the transverse direction and the loop flattens;
wherein the device further comprises a plurality of strip-shaped mechanical fastening tabs, wherein a single tab is provided for each eyelet or ring, as applicable, each tab comprising a proximal end portion and a distal end portion being connected by an inner tab portion, wherein said proximal end portion is releasably or fixedly attached to the first lateral edge region of sleeve such that the tab is located opposite to an eyelet or ring, as applicable, and extends in substantially the transverse direction of the sleeve with its distal end portion positioned away from the central portion of the sleeve, wherein each tab has a first major surface located towards the outer surface of the sleeve and a second major surface located away from the outer surface of the sleeve, wherein the second major surface at the distal end portion of the tab comprises one part of a mechanical fastening system and said second major surface at the proximal end portion of the tab comprise the complementary part of the mechanical fastening system; and wherein the tabs and eyelets or rings, as applicable, are configured and arranged such that, in use, the tabs are passed through the eyelets or rings, as applicable, then turned back on themselves such that the first lateral side edge of the sleeve is drawn towards the eyelets or rings, as applicable, and then fastened so that the sleeve is tightened and restrained about the limb of the user.

7. A compression device according to claim 6, wherein the gusset either is in at least part integral with the adjacent-lying material of the sleeve or is provided as an inset into the sleeve.

8. A compression device according to any one of claims 6 to 7, wherein the gusset comprises two layers, an outer layer of material and an inner layer of material, the product of the modulus of elasticity of the inner-layer-material times the thickness of the inner-layer-material being less than the product of the modulus of elasticity of the outer-layer-material times the thickness of the outer-layer-material, wherein outer-layer-material is said loop material and wherein the inner layer of material is affixed to the outer layer of material, so as to provide a loop of outer-layer-material above the inner layer when the gusset is in its non-expanded state, which, in use under the provision of tension and accordingly expansion of the gusset in the transverse direction, can flatten.

9. A compression device according to any one of claims 6 to 7, wherein the gusset comprises two layers, an outer layer of material and an inner layer of material, the product of the modulus of elasticity of the inner-layer-material times the thickness of the inner-layer-material being less than the product of the modulus of elasticity of the outer-layer-material times the thickness of the outer-layer-material, wherein the inner layer of material and outer layer of material are configured and arranged, so as to provide:
at least one loop of outer-layer-material above the inner layer when the gusset is in its non-expanded state which, in use under the provision of tension and accordingly expansion of the gusset in the transverse direction, can flatten;
and at least one region of outer-layer material above the inner layer, where the outer-layer material includes a slit extending substantially lengthwise relative to said upper and lower edges of the sleeve (slit-region), so that when the gusset is in its non-expanded state, the outer-layer material in said at least one slit-region lies substantially flat with the lateral edges of said slit near to or overlapping one another, and in use, under the provision of tension and accordingly expansion of expandable portion of the gusset in the transverse direction, the lateral edges of the said slit move apart exposing the underlying inner layer.

10. A compression device according to claim 9, wherein the inner layer is provided with indicia that become visible when the lateral edges of the slit move apart and/or the inner layer is provided with a color that is different to the color of the outer-layer.

11. A compression device according to any one of claims 6 to 10, wherein in use on the limb of the user, the sleeve is disposed about a central axis (A), said central axis lying in a plane (P), and the gusset extends along a second axis (G), wherein relative to a projection of the second axis onto said plane (P) containing the central axis (A), the second axis (G) is either in parallel alignment or inclined forming an acute angle (β) up to 25° inclusive relative to the central axis.

12. A compression device according to any one of claims 6 to 11, wherein the compression device comprises rings, wherein each ring is attached by a strap extending between the sleeve and the ring and wherein the rings are positioned along the second lateral edge of the sleeve either adjacent to or spaced apart from said edge and away from the second lateral edge portion.

13. A compression device according to any one of the preceding claims, wherein the first lateral edge region of the sleeve is trimmable.

14. A compression device according to claim 13 as dependent directly or indirectly on claim 5, wherein the first lateral edge region, the central region and the second lateral edge region of the sleeve have a structure or is provided with a structure that is adapted to be engaged by said second tab fasteners and strap fasteners, and wherein the first and second lateral edge regions of the sleeve are trimmable.

## Patentansprüche

1. Eine Kompressionsvorrichtung zum Anlegen von Kompression an eine Gliedmaße eines Benutzers, umfassend eine Hülse, im Wesentlichen zum Abdecken eines Abschnitts der Gliedmaße eines Benutzers, wobei die Hülse eine Außenoberfläche, eine Innenoberfläche, einen oberen Rand, einen unteren Rand und zwei Seitenränder aufweist, wobei in der Querrichtung von dem ersten Seitenrand zu dem zweiten Seitenrand die Hülse einen ersten Seitenbereich, einen Mittelbereich und einen zweiten Seitenbereich aufweist, wobei der zweite Seitenrandbereich der Hülse zwischen dem oberen und unteren Rand der Hülse mit einer Mehrzahl von Ringen in Reihe bereitgestellt ist, wobei jeder Ring lösbar oder fest durch einen Riemen angegliedert ist, der sich zwischen der Hülse und dem Ring im Wesentlichen in der Querrichtung der Hülse erstreckt, wobei mindestens ein Abschnitt des Riemens mindestens in Querrichtung expandierbar ist, wobei der expandierbare Abschnitt ein Material mit Elastizität in mindestens der Querrichtung umfasst und konfiguriert und angeordnet ist, sodass, wenn der expandierbare Abschnitt in seinem nicht-expandierten Zustand vorliegt, es außerhalb eine nach außen hin ansteigende Materialschleife gibt, und wobei bei Verwendung unter der Bereitstellung von Spannung in der Querrichtung der Hülse der expandierbare Abschnitt sich in der Querrichtung erstreckt und die Schleife abflacht;
wobei die Vorrichtung ferner eine Mehrzahl von streifenförmigen mechanischen Befestigungslaschen umfasst, wobei eine einzige Lasche für jeden Ring bereitgestellt ist, jede Lasche einen proximalen Endabschnitt und einen distalen Endabschnitt umfasst, die durch einen inneren Laschenabschnitt verbunden sind, wobei der proximale Endabschnitt lösbar oder fest an dem ersten Seitenrandbereich der Hülse angegliedert ist, sodass sich die Lasche einem Ring gegenüberliegend befindet und sich im Wesentlichen in die Querrichtung der Hülse erstreckt, mit seinem distalen Endabschnitt weg von dem Mittelteil der Hülse positioniert, wobei jede Lasche eine erste Hauptoberfläche, die sich in Richtung der Außenoberfläche der Hülse befindet und eine zweite Hauptfläche, die sich weg von der Außenoberfläche der Hülse befindet, aufweist, wobei die zweite Hauptoberfläche an dem distalen Endabschnitt der Lasche einen Teil eines mechanischen Befestigungssystems umfasst und die zweite Hauptoberfläche an dem proximalen Endabschnitt der Lasche den komplementären Teil des mechanischen Befestigungssystems umfasst; und wobei die Laschen und Ringe konfiguriert und angeordnet sind, sodass, bei Verwendung die Laschen durch die Ringe geführt auf sich selbst zurückgedreht werden, sodass der erste Seitenrand der Hülse in Richtung der Ringe gezogen und anschließend befestigt wird, sodass die Hülse angezogen und um die Gliedmaße des Benutzers herum zurückgehalten wird.

2. Eine Kompressionsvorrichtung nach einem der vorhergehenden Ansprüche, wobei der expandierbare Abschnitt des Riemens zwei Schichten umfasst, eine Außenmaterialschicht und eine Innenmaterialschicht, wobei das Produkt des Elastizitätsmoduls des Innenschichtmaterials mal die Dicke des Innenschichtmaterials weniger beträgt als das Produkt des Elastizitätsmoduls des Außenschichtmaterials mal die Dicke des Außenschichtmaterials, wobei das Außenschichtmaterial das Schleifenmaterial ist und wobei die Innenmaterialschicht an der Außenmaterialschicht fixiert ist, um eine Schleife von Außenschichtmaterial oberhalb der Innenschicht bereitzustellen, wenn der expandierbare Riemen in seinem nicht expandierten Zustand vorliegt, die, bei Verwendung unter der Bereitstellung von Spannung und entsprechend Expansion des expandierbaren Abschnitts des Riemens in der Querrichtung, abflachen kann.

3. Eine Kompressionsvorrichtung nach einem der Ansprüche 1 bis 2, wobei der expandierbare Abschnitt des Riemens zwei Schichten umfasst, eine Außenmaterialschicht und eine Innenmaterialschicht, wobei das Produkt des Elastizitätsmoduls des Innenschichtmaterials mal die Dicke des Innenschichtmaterials weniger beträgt als das Produkt des Elastizitätsmoduls des Außenschichtmaterials mal die Dicke des Außenschichtmaterials, wobei die Innenmaterialschicht und die Außenmaterialschicht konfiguriert und angeordnet sind, um Folgendes bereitzustellen:
mindestens eine Schleife aus Außenschichtmaterial oberhalb der Innenschicht, wenn der expandierbare Riemen in seinem nicht expandierten Zustand vorliegt, die bei Verwendung unter der Bereitstellung von Spannung und somit der Expansion des expandierbaren Abschnitts des Riemens in der Querrichtung, abflachen kann; und
mindestens einen Bereich aus Außenschichtmaterial oberhalb der Innenschicht, wobei das Außenschichtmaterial einen Schlitz beinhaltet, der sich im Wesentlichen in Längsrichtung relativ zu dem oberen und dem unteren Rand der Hülse (Schlitzbereich) erstreckt, sodass, wenn der expandierbare Riemenabschnitt in seinem nicht expandierten Zustand vorliegt, das Außenschichtmaterial in dem mindestens einen Schlitzbereich im Wesentlichen flach mit den Seitenrändern des Schlitzes nahe an oder einander überlappend liegt und bei Verwendung, unter der Bereitstellung von Spannung und somit Expansion des expandierbaren Abschnitts des Riemens in der Querrichtung, die Seitenränder des Schlitzes sich auseinander bewegen und die darunter liegende Innenschicht freilegen.

4. Eine Kompressionsvorrichtung nach Anspruch 3, wobei die Innenschicht mit Zeichen bereitgestellt ist, die sichtbar werden, wenn die Seitenränder des Schlitzes sich auseinander bewegen und/oder die Innenschicht mit einer Farbe bereitgestellt ist, die sich von der Farbe der Außenschicht unterscheidet.

5. Eine Kompressionsvorrichtung nach Anspruch 1, wobei, wenn die Abschnitte der Riemen in ihrem nicht expandierten Zustand vorliegen, die Ringe von dem zweiten Seitenrand in Richtung zum Mittelbereich beabstandet sind, wobei insbesondere jeder Ring einen Seitenrand aufweist, von dem Mittelteil der Hülse distanziert, wobei der Seitenrand des Rings von dem zweiten Seitenrand in einem Abstand zwischen 3 und 25 cm, einschließlich der Endpunkte, beabstandet ist, und wobei die Ringe lösbar an der Außenoberfläche an dem zweiten Seitenrandbereich angegliedert sind, wobei jeder Riemen ferner einen proximalen Endabschnitt umfasst, wobei die Innenoberfläche am proximalen Endabschnitt des Riemens mit dem Haken, Schaft und/oder becherförmigen Befestigungselementen (Riemenbefestigungsmittel) bereitgestellt ist, und wobei die Außenoberfläche an dem zweiten Seitenrandbereich der Hülse eine Struktur aufweist oder mit einer Struktur bereitgestellt ist, die angepasst ist, um durch die Riemenbefestigungsmittel in Eingriff gebracht zu werden.

6. Eine Kompressionsvorrichtung zum Anlegen von Kompression an eine Gliedmaße eines Benutzers, umfassend eine Hülse, im Wesentlichen zum Abdecken eines Abschnitts der Gliedmaße eines Benutzers, wobei die Hülse eine Außenoberfläche, eine Innenoberfläche, einen oberen Rand, einen unteren Rand und zwei Seitenränder aufweist, wobei in der Querrichtung von dem ersten Seitenrand zu dem zweiten Seitenrand die Hülse einen ersten Seitenbereich, einen Mittelbereich und einem zweiten Seitenbereich aufweist, wobei der zweite seitliche Randbereich der Hülse entweder mit einer Mehrzahl von Ösen oder einer Mehrzahl von Ringen in Reihe zwischen dem oberen und dem unteren Rand der Hülse bereitgestellt ist, wobei die Hülse einen länglichen, expandierbaren Zwickel beinhaltet, der sich im Wesentlichen in Längsrichtung zwischen dem oberen und dem unteren Rand der Hülse erstreckt, wobei der Zwickel in mindestens der Querrichtung der Hülse expandierbar ist, wobei der expandierbare Zwickel ein Material mit Elastizität in mindestens der Querrichtung umfasst und konfiguriert und angeordnet ist, sodass, wenn der expandierbare Zwickel in seinem nicht expandierten Zustand vorliegt, es außerhalb eine nach außen ansteigende Materialschleife gibt, und wobei, bei Verwendung unter Bereitstellung von Spannung in der Querrichtung der Hülse, der expandierbare Zwickel in die Querrichtung expandiert und die Schleife abflacht;
wobei die Vorrichtung ferner eine Mehrzahl von streifenförmigen mechanischen Befestigungslaschen umfasst, wobei eine einzelne Lasche für jede Öse oder gegebenenfalls für jeden Ring bereitgestellt ist, wobei jede Lasche einen proximalen Endabschnitt und einen distalen Endabschnitt umfasst, die durch eine inneren Laschenabschnitt verbunden sind, wobei der proximale Endabschnitt lösbar oder fest an dem ersten Seitenrandbereich der Hülse angegliedert ist, sodass sich die Lasche einer Öse oder gegebenenfalls eines Rings gegenüberliegend befindet, und sich im Wesentlichen in der Querrichtung der Hülse mit ihrem distalen Ende weg von dem Mittelteil der Hülse erstreckt, wobei jede Lasche eine erste Hauptoberfläche, die sich in Richtung der Außenoberfläche der Hülse befindet, und eine zweite Hauptoberfläche, die sich von der Außenoberfläche der Hülse weg befindet, aufweist, wobei die zweite Hauptoberfläche an dem distalen Endabschnitt der Lasche einen Teil eines mechanischen Befestigungssystems umfasst und die zweite Hauptoberfläche an dem proximalen Endabschnitt der Lasche den komplementären Teil des mechanischen Befestigungssystems umfasst; und wobei die Laschen und Ösen oder gegebenenfalls Ringe konfiguriert und angeordnet sind, sodass bei Verwendung die Laschen durch die Ösen oder gegebenenfalls Ringe geführt werden, anschließend auf sich selbst zurückgedreht werden, sodass der erste Seitenrand der Hülse in Richtung der Ösen oder gegebenenfalls Ringe gezogen und anschließend so befestigt wird, dass die Hülse angezogen wird und um die Gliedmaße des Benutzers herum zurückgehalten wird.

7. Eine Kompressionsvorrichtung nach Anspruch 6, wobei der Zwickel entweder mindestens teilweise mit dem angrenzend liegenden Material der Hülse einstückig ist oder als ein Einsatz in die Hülse bereitgestellt ist.

8. Eine Kompressionsvorrichtung nach einem der Ansprüche 6 bis 7, wobei der Zwickel zwei Schichten umfasst, eine Außenmaterialschicht und eine Innenmaterialschicht, wobei das Produkt des Elastizitätsmoduls des Innenschichtmaterials mal die Dicke des Innenschichtmaterials weniger beträgt als das Produkt des Elastizitätsmoduls des Außenschichtmaterials mal die Dicke des Außenschichtmaterials, wobei das Außenschichtmaterial das Schleifenmaterial ist und wobei das Innenschichtmaterial an dem Außenschichtmaterial angebracht ist, um eine Schleife aus Außenschichtmaterial oberhalb der Innenschicht bereitzustellen, wenn der Zwickel in seinem nicht expandierten Zustand vorliegt, der bei Verwendung unter der Bereitstellung von Spannung und somit Expansion des Zwickels in der Querrichtung abflachen kann.

9. Eine Kompressionsvorrichtung nach einem der Ansprüche 6 bis 7, wobei der Zwickel zwei Schichten umfasst, eine Außenmaterialschicht und eine Innenmaterialschicht, wobei das Produkt des Elastizitätsmoduls des Innenschichtmaterials mal die Dicke des Innenschichtmaterials weniger beträgt als das Produkt des Elastizitätsmoduls des Außenschichtmaterials mal die Dicke des Außenschichtmaterials, wobei die Innenmaterialschicht und die Außenmaterialschicht konfiguriert und angeordnet sind, um Folgendes bereitzustellen:
mindestens eine Schleife aus Außenschichtmaterial oberhalb der Innenschicht, wenn der Zwickel in seinem nicht-expandierten Zustand vorliegt, die bei Verwendung unter der Bereitstellung von Spannung und somit Expansion des Zwickels in der Querrichtung abflachen kann;
und mindestens einen Bereich aus Außenschichtmaterial oberhalb der Innenschicht, wobei das Außenschichtmaterial einen Schlitz beinhaltet, der sich im Wesentlichen in Längsrichtung relativ zu dem oberen und dem unteren Rand der Hülse (Schlitzbereich) erstreckt, sodass, wenn der Zwickel in seinem nicht-expandierten Zustand vorliegt, das Außenschichtmaterial in dem mindestens einen Schlitzbereich im Wesentlichen flach mit der Seitenrändern des Schlitzes nahe an oder einander überlappend liegt, und bei Verwendung unter der Bereitstellung von Spannung und somit Expansion des expandierbaren Abschnitts des Zwickels in der Querrichtung sich die Seitenränder des Schlitzes auseinander bewegen, die darunter liegende Innenschicht freilegend.

10. Eine Kompressionsvorrichtung nach Anspruch 9, wobei die Innenschicht mit Zeichen bereitgestellt ist, die sichtbar werden, wenn sich die Seitenränder des Schlitzes auseinander bewegen und/oder die Innenschicht mit einer Farbe bereitgestellt ist, die von der Farbe der Außenschicht verschieden ist.

11. Eine Kompressionsvorrichtung nach einem der Ansprüche 6 bis 10, wobei bei Verwendung an der Gliedmaße des Benutzers die Hülse um eine Mittelachse (A) angebracht ist, wobei die Mittelachse in einer Ebene (P) liegt und der Zwickel sich entlang einer zweiten Achse (G) erstreckt, wobei, relativ zu einer Projektion der zweiten Achse auf die Ebene (P), die die Mittelachse (A) enthält, die zweite Achse (G) entweder parallel ausgerichtet oder geneigt ist, relativ zu der Mittelachse einen spitzen Winkel (β) bis zu einschließlich 25° bildend.

12. Eine Kompressionsvorrichtung nach einem der Ansprüche 6 bis 11, wobei die Kompressionsvorrichtung Ringe umfasst, wobei jeder Ring durch einen Riemen angegliedert ist, der sich zwischen der Hülse und dem Ring erstreckt, und wobei die Ringe entlang des zweiten Seitenrands der Hülse entweder benachbart oder beabstandet von dem Rand und weg von dem zweiten Seitenrandabschnitt positioniert sind.

13. Eine Kompressionsvorrichtung nach einem der vorstehenden Ansprüche, wobei der erste Seitenrandbereich der Hülse trimmbar ist.

14. Eine Kompressionsvorrichtung nach Anspruch 13, wie direkt oder indirekt abhängig von Anspruch 5, wobei der erste Seitenrandbereich, der Mittelbereich und der zweite Seitenrandbereich der Hülse eine Struktur aufweist oder mit einer Struktur bereitgestellt ist, die angepasst ist, um durch die zweiten Laschenbefestigungen und Riemenbefestigungen in Eingriff gebracht zu werden, und wobei der erste und der zweite Seitenrandbereich der Hülse trimmbar sind.

## Revendications

1. Dispositif de compression pour appliquer une compression à un membre d'un utilisateur comprenant une gaine pour couvrir sensiblement une partie du membre d'un utilisateur, dans lequel la gaine a une surface externe, une surface interne, un bord supérieur, un bord inférieur et deux bords latéraux, dans lequel dans la direction transversale allant du premier bord latéral au deuxième bord latéral la gaine comprend une première région latérale, une région centrale et une deuxième région latérale, dans lequel la deuxième région de bord latéral de la gaine est pourvue d'une pluralité d'anneaux en série entre les bords supérieur et inférieur de la gaine, chaque anneau étant fixé de manière amovible ou fixe par une sangle s'étendant entre la gaine et l'anneau essentiellement dans la direction transversale de la gaine, au moins une partie de ladite sangle étant expansible au moins dans la direction transversale, dans lequel ladite partie expansible comprend un matériau ayant une élasticité au moins dans la direction transversale et est configurée et agencée, de telle sorte que lorsque la partie expansible est dans son état non expansé il y a à l'extérieur une boucle de matériau dépassant vers l'extérieur et lorsque, en cours d'utilisation on fournit une tension dans la direction transversale de la gaine, la partie expansible subit une expansion dans la direction transversale et la boucle s'aplatit ;
dans lequel le dispositif comprend en outre une pluralité de languettes de fixation mécanique en forme de bande, dans lequel une unique languette est fournie pour chaque anneau, chaque languette comprenant une partie d'extrémité proximale et une partie d'extrémité distale étant reliées par une partie de languette interne, dans lequel ladite partie d'extrémité proximale est fixée de manière libérable ou fixe à la première région de bord latéral de la gaine de telle sorte que la languette est située opposée à un anneau et s'étend essentiellement dans la direction transversale de la gaine, avec sa partie d'extrémité distale positionnée à l'écart de la partie centrale de la gaine, dans lequel chaque languette a une première surface principale située en direction de la surface externe de la gaine et une deuxième surface principale située à l'écart de la surface externe de la gaine, dans lequel la deuxième surface principale au niveau de la partie d'extrémité distale de la languette comprend une partie d'un système de fixation mécanique et ladite deuxième surface principale au niveau de la partie d'extrémité proximale de la languette comprend la partie complémentaire du système de fixation mécanique ; et dans lequel les languettes et anneaux sont configurés et agencés de telle sorte que, en cours d'utilisation, les languettes sont passées à travers les anneaux, retournées sur elles-mêmes de telle sorte que le premier bord latéral de la gaine est tiré en direction des anneaux puis fixé de sorte que la gaine est serrée et contrainte autour du membre de l'utilisateur.

2. Dispositif de compression selon l'une quelconque des revendications précédentes, dans lequel la partie expansible de la sangle comprend deux couches, une couche externe de matériau et une couche interne de matériau, le produit du module d'élasticité du matériau de couche interne fois l'épaisseur du matériau de couche interne étant inférieur au produit du module d'élasticité du matériau de couche externe fois l'épaisseur du matériau de couche externe, dans lequel le matériau de couche externe est ledit matériau de boucle et dans lequel la couche interne de matériau est attachée à la couche externe de matériau, de façon à fournir une boucle de matériau de couche externe au-dessus de la couche interne lorsque la partie de sangle expansible est dans son état non expansé, qui, en cours d'utilisation lorsqu'on fournit une tension et, en conséquence, une expansion de la partie expansible de la sangle dans la direction transversale, peut s'aplatir.

3. Dispositif de compression selon l'une quelconque des revendications 1 à 2, dans lequel la partie expansible de la sangle comprend deux couches, une couche externe de matériau et une couche interne de matériau, le produit du module d'élasticité du matériau de couche interne fois l'épaisseur du matériau de couche interne étant inférieur au produit du module d'élasticité du matériau de couche externe fois l'épaisseur du matériau de couche externe, dans lequel la couche interne de matériau et la couche externe de matériau sont configurées et agencées, de façon à fournir :
au moins une boucle de matériau de couche externe au-dessus de la couche interne lorsque la partie de sangle expansible est dans son état non expansé qui, en cours d'utilisation lorsqu'on fournit une tension et, en conséquence, une expansion de la partie expansible de la sangle dans la direction transversale, peut s'aplatir ; et
au moins une région de matériau de couche externe au-dessus de la couche interne, où le matériau de couche externe inclut une fente s'étendant de façon essentiellement longitudinale par rapport auxdits bords supérieur et inférieur de la gaine (région de fente), de sorte que lorsque la partie de sangle expansible est dans son état non expansé, le matériau de couche externe dans ladite au moins une région de fente se trouve essentiellement à plat avec les bords latéraux de ladite fente l'un près de l'autre ou se chevauchant et en cours d'utilisation, lorsqu'on fournit une tension et, en conséquence, une expansion de la partie expansible de la sangle dans la direction transversale, les bords latéraux de ladite fente se séparent en exposant la couche interne sous-jacente.

4. Dispositif de compression selon la revendication 3, dans lequel la couche interne est pourvue d'éléments visuels qui deviennent visibles lorsque les bords latéraux de la fente se séparent et/ou la couche interne est pourvue d'une couleur qui est différente de la couleur de la couche externe.

5. Dispositif de compression selon la revendication 1, dans lequel, lorsque lesdites parties des sangles sont dans leur état non expansé, les anneaux sont espacés du deuxième bord latéral en direction de la région centrale, en particulier dans lequel chaque anneau a un bord latéral distant de la partie centrale de la gaine, ledit bord latéral de l'anneau est espacé du deuxième bord latéral à une distance comprise entre 3 et 25 cm, points de terminaison inclus, et dans lequel les anneaux sont fixés de façon libérable à la surface externe au niveau de la deuxième région de bord latéral, dans lequel chaque sangle comprend en outre une partie d'extrémité proximale, dans lequel la surface interne au niveau de la partie d'extrémité proximale de la sangle est pourvue de fixations à crochet, à tige et/ou en forme de coupelle (fixations de sangle) et dans lequel la surface externe au niveau de la deuxième région de bord latéral de la gaine a une structure ou est pourvue d'une structure qui est conçue pour être mise en prise par lesdites fixations de sangle.

6. Dispositif de compression pour appliquer une compression à un membre d'un utilisateur comprenant une gaine pour couvrir sensiblement une partie du membre d'un utilisateur, dans lequel la gaine a une surface externe, une surface interne, un bord supérieur, un bord inférieur et deux bords latéraux, dans lequel dans la direction transversale allant du premier bord latéral au deuxième bord latéral la gaine comprend une première région latérale, une région centrale et une deuxième région latérale, dans lequel la deuxième région de bord latéral de la gaine est pourvue soit d'une pluralité d'oeillets soit d'une pluralité d'anneaux en série entre les bords supérieur et inférieur de la gaine, dans lequel la gaine inclut un soufflet expansible allongé s'étendant de façon sensiblement longitudinale entre les bords supérieur et inférieur de la gaine, ledit soufflet étant expansible au moins dans la direction transversale de la gaine, dans lequel ledit soufflet expansible comprend un matériau ayant une élasticité au moins dans la direction transversale et est configuré et agencé, de telle sorte que lorsque le soufflet expansible est dans son état non expansé il y a à l'extérieur une boucle de matériau dépassant vers l'extérieur, et lorsque, en cours d'utilisation on fournit une tension dans la direction transversale de la gaine, le soufflet expansible subit une expansion dans la direction transversale et la boucle s'aplatit ;
dans lequel le dispositif comprend en outre une pluralité de languettes de fixation mécanique en forme de bande, dans lequel une unique languette est fournie pour chaque oeillet ou anneau, selon ce qui est applicable, chaque languette comprenant une partie d'extrémité proximale et une partie d'extrémité distale étant reliées par une partie de languette interne, dans lequel ladite partie d'extrémité proximale est fixée de manière libérable ou fixe à la première région de bord latéral de la gaine de telle sorte que la languette se situe opposée à un oeillet ou anneau, selon ce qui est applicable, et s'étend essentiellement dans la direction transversale de la gaine, avec sa partie d'extrémité distale positionnée à l'écart de la partie centrale de la gaine, dans lequel chaque languette a une première surface principale située en direction de la surface externe de la gaine et une deuxième surface principale située à l'écart de la surface externe de la gaine, dans lequel la deuxième surface principale au niveau de la partie d'extrémité distale de la languette comprend une partie d'un système de fixation mécanique et ladite deuxième surface principale au niveau de la partie d'extrémité proximale de la languette comprend la partie complémentaire du système de fixation mécanique ; et dans lequel les languettes et oeillets ou anneaux, selon ce qui est applicable, sont configurés et agencés de telle sorte que, en cours d'utilisation, les languettes sont passées à travers les oeillets ou anneaux, selon ce qui est applicable, puis retournées sur elles-mêmes de telle sorte que le premier bord latéral de la gaine est tiré en direction des oeillets ou anneaux, selon ce qui est applicable, puis fixé de sorte que la gaine est serrée et contrainte autour du membre de l'utilisateur.

7. Dispositif de compression selon la revendication 6, dans lequel le soufflet est soit au moins en partie solidaire du matériau de la gaine se trouvant à côté soit est fourni en tant qu'insert dans la gaine.

8. Dispositif de compression selon l'une quelconque des revendications 6 à 7, dans lequel le soufflet comprend deux couches, une couche externe de matériau et une couche interne de matériau, le produit du module d'élasticité du matériau de couche interne fois l'épaisseur du matériau de couche interne étant inférieur au produit du module d'élasticité du matériau de couche externe fois l'épaisseur du matériau de couche externe, dans lequel le matériau de couche externe est ledit matériau de boucle et dans lequel la couche interne de matériau est attachée à la couche externe de matériau, de façon à fournir une boucle de matériau de couche externe au-dessus de la couche interne lorsque le soufflet est dans son état non expansé, qui, en cours d'utilisation lorsqu'on fournit une tension et, en conséquence, une expansion du soufflet dans la direction transversale, peut s'aplatir.

9. Dispositif de compression selon l'une quelconque des revendications 6 à 7, dans lequel le soufflet comprend deux couches, une couche externe de matériau et une couche interne de matériau, le produit du module d'élasticité du matériau de couche interne fois l'épaisseur du matériau de couche interne étant inférieur au produit du module d'élasticité du matériau de couche externe fois l'épaisseur du matériau de couche externe, dans lequel la couche interne de matériau et la couche externe de matériau sont configurées et agencées, de façon à fournir :
au moins une boucle de matériau de couche externe au-dessus de la couche interne lorsque le soufflet est dans son état non expansé qui, en cours d'utilisation lorsqu'on fournit une tension et, en conséquence, une expansion du soufflet dans la direction transversale, peut s'aplatir ;
et au moins une région de matériau de couche externe au-dessus de la couche interne, où le matériau de couche externe inclut une fente s'étendant de façon essentiellement longitudinale par rapport auxdits bords supérieur et inférieur de la gaine (région de fente), de sorte que lorsque le soufflet est dans son état non expansé, le matériau de couche externe dans ladite au moins une région de fente se trouve essentiellement à plat avec les bords latéraux de ladite fente l'un près de l'autre ou se chevauchant, et en cours d'utilisation, lorsqu'on fournit une tension et, en conséquence, une expansion de la partie expansible du soufflet dans la direction transversale, les bords latéraux de ladite fente se séparent exposant la couche interne sous-jacente.

10. Dispositif de compression selon la revendication 9, dans lequel la couche interne est pourvue d'éléments visuels qui deviennent visibles lorsque les bords latéraux de la fente se séparent et/ou la couche interne est pourvue d'une couleur qui est différente de la couleur de la couche externe.

11. Dispositif de compression selon l'une quelconque des revendications 6 à 10, dans lequel en cours d'utilisation sur le membre de l'utilisateur, la gaine est disposée autour d'un axe central (A), ledit axe central se trouvant dans un plan (P), et le soufflet s'étend le long d'un deuxième axe (G), dans lequel par rapport à une projection du deuxième axe sur ledit plan (P) contenant l'axe central (A), le deuxième axe (G) est soit en alignement parallèle soit incliné en formant un angle aigu (β) jusqu'à 25° inclus par rapport à l'axe central.

12. Dispositif de compression selon l'une quelconque des revendications 6 à 11, dans lequel le dispositif de compression comprend des anneaux, dans lequel chaque anneau est fixé par une sangle s'étendant entre la gaine et l'anneau et dans lequel les anneaux sont positionnés le long du deuxième bord latéral de la gaine soit adjacents audit bord soit espacés de celui-ci et à l'écart de la deuxième partie de bord latéral.

13. Dispositif de compression selon l'une quelconque des revendications précédentes, dans lequel la première région de bord latéral de la gaine est ajustable.

14. Dispositif de compression selon la revendication 13 dépendant directement ou indirectement de la revendication 5, dans lequel la première région de bord latéral, la région centrale et la deuxième région de bord latéral de la gaine ont une structure ou sont pourvues d'une structure qui est conçue pour être mise en prise par lesdites deuxièmes fixations à languette et fixations de sangle, et dans lequel les première et deuxième régions de bord latéral de la gaine sont ajustables.
